# EUROPÄISCHE PATENTANMELDUNG

(11) **EP 3 936 162 A2**
(43) Veröffentlichungstag der Anmeldung: **12.01.2022**
(21) Anmeldenummer: 21180233.5
(22) Anmeldetag: 18.06.2021
(51) Int. Cl.: A61L 9/20, C02F 1/32, F21V 7/00

(54) **BESTRAHLUNGSVORRICHTUNG ZUR UV-BESTRAHLUNG EINES STRÖMENDEN MEDIUMS**

(30) Priorität: 19.06.2020 EP 20181097
(71) Anmelder: Virobuster International GmbH, 53578 Windhagen (DE)
(72) Erfinder: Yigit, Fehmi, 48599 Gronau (DE)
(74) Vertreter: Weidener, Jörg Michael

(57) **Zusammenfassung**

Die Erfindung betrifft Bestrahlungsvorrichtung (1) zur UV-Bestrahlung, insbesondere UV-C-Bestrahlung, eines die Bestrahlungsvorrichtung (1) durchströmenden Mediums, insbesondere Wasser oder ein gasförmiges Medium, bevorzugt Luft, insbesondere zur Inaktivierung von in dem Medium befindlichen Mikroorganismen, wie Bakterien, Keime, Schimmel und/oder Viren, mit einem einen Einlass (2) und einen Auslass (3) für das Medium aufweisenden zu durchströmenden Gehäuse (4) und einer Mehrzahl von im Inneren des Gehäuses (4) angeordneten, UV-Strahlung emittierenden Strahlungsquellen (5) zur Bestrahlung des das Gehäuse (4) durchströmenden Mediums, wobei das Gehäuse (4) einen Reflektor (21) aufweist, wobei der Reflektor (21) an der der Strahlungsquelle (5) zugewandten Innenseite (6) zumindest bereichsweise, vorzugsweise vollflächig, reflektierend einen Reflexionsgrad für die von der Strahlungsquelle (5) emittierten UV-Strahlung von wenigstens 0,6 aufweist, wobei die wenigstens eine Strahlungsquelle (5) mittels einer Halteeinrichtung (22) gehalten und/oder fixiert ist, wobei die Halteeinrichtung (22), vorzugsweise lösbar, mit dem Gehäuse (4) und/oder dem Reflektor (21) verbunden ist. Erfindungsgemäß ist vorgesehen, dass die Halteeinrichtung (22) eine erste Halteeinheit (23) für die Strahlungsquellen (5) aufweist, dass wenigstens zwei Strahlungsquellen (5) derart in dem und/oder an der ersten Halteeinheit (23) angeordnet und/oder gelagert sind, dass die minimalen Abstände der Strahlungsquellen (5) zu der Innenseite des Reflektors (21) sich voneinander unterscheiden. In einer weiteren Erfindung ist die erste Halteeinheit (23) über ein erstes Verbindungsmittel (24) der Halteeinrichtung (22) mit dem Gehäuse (4) lösbar verbindbar ist, wobei die erste Halteeinheit (23) wenigstens drei zueinander zumindest bereichsweise beabstandete, als stegförmige und/oder langgestreckte Haltearme ausgebildete erste Haltemittel (25) aufweist, wobei die ersten Haltemittel (25) mit einem Endbereich (28) an einem ersten, mit dem Verbindungsmittel (24) verbundenen Verbindungsbereich (26) gehalten sind und wobei die Strahlungsquellen (5), vorzugsweise jeweils an einem stirnseitigen Endbereich (27), mit den ersten Haltemitteln (25), vorzugsweise lösbar und formschlüssig, kraftschlüssig und/oder reibschlüssig, an dem anderen, insbesondere freien, Endbereich (29) der ersten Haltemittel (25) verbunden sind.

## Beschreibung

Die Erfindung betrifft eine Bestrahlungsvorrichtung zur UV-Bestrahlung, insbesondere UV-C-Bestrahlung, eines die Bestrahlungsvorrichtung durchströmenden Mediums, insbesondere Wasser, Luft und/oder ein Gasgemisch und/oder ein Dampfgemisch, insbesondere zur Inaktivierung und/oder Abtötung von in dem Medium befindlichen Mikroorganismen, wie Bakterien, Keime, Schimmel und/oder Viren. Die Bestrahlungsvorrichtung weist einen Einlass und einen Auslass für das Medium bzw. den Mediumstrom auf, wobei der Einlass und der Auslass an einem von dem Medium durchströmten Gehäuse vorgesehen sind. Im Inneren des Gehäuses ist eine UV-Strahlung, insbesondere UV-C-Strahlung, emittierende Strahlungsquelle zur Bestrahlung des das Gehäuses durchströmenden Mediums angeordnet.

Insbesondere betrifft die vorliegende Erfindung das technische Gebiet der sogenannten UV-Desinfektion. Der Begriff "UV-Desinfektion" bezeichnet Verfahren, bei denen Mikroorganismen - auch als Mikroben bezeichnet - durch die Behandlung mit UV-Strahlung abgetötet werden können. Dabei kann die UV-Desinfektion zur Trinkwasseraufbereitung, zur Oberflächendesinfizierung, zur Abluftaufbereitung aber auch im Bereich der hygienischen Verarbeitung von Lebensmitteln oder dergleichen eingesetzt werden. Auch Luft in öffentlichen Bereichen kann so "sauber" - das heißt eine geringe Belastung mit schädlichen Mikroorganismen aufweisend - gehalten werden.

Als UV-C-Strahlung wird nachfolgend eine Strahlung mit einer Wellenlänge zwischen 100 nm bis 280 nm verstanden, insbesondere wobei im Rahmen der UV-Desinfektion UV-C-Strahlung bei einer Wellenlänge zwischen 200 nm bis 280 nm, vorzugsweise 240 nm bis 280 nm, eingesetzt wird.

Die UV-Desinfektion nutzt insbesondere eine Wellenlänge der UV-Strahlung zwischen 200 bis 300 nm. Dabei wirkt die abgegebene UV-Strahlung bakterizid - das heißt sie wird von der DNA absorbiert und zerstört deren Struktur und inaktiviert lebende Zellen. So können Mikroorganismen, wie Viren, Bakterien, Hefen und Pilze, mit UV-Strahlung innerhalb kürzester Zeit, insbesondere innerhalb von wenigen Sekunden, unschädlich gemacht werden.

Bei ausreichend hoher Bestrahlungsstärke ist die UV-Desinfektion somit eine zuverlässige und ökologische Methode, da insbesondere keine Zugabe von weiteren Chemikalien notwendig ist. Besonders vorteilhaft ist, dass Mikroorganismen keine Resistenz gegen UV-Strahlung entwickeln können. Letztlich kann durch die UV-Desinfektion auch die Vermehrung der Mikroorganismen unterbrochen werden.

Das Verfahren der UV-Desinfektion kann auch als "Ultraviolet Germicidal Irritation" (UVGI) und/oder als Mikroben-Desinfektion bezeichnet werden, insbesondere wobei UV-Strahlung mit einer Wellenlänge von 254 nm eingesetzt wird. Der Einsatz der UV-Desinfektion ist zur Virusinaktivierung bei der Luftreinigung besonders vorteilhaft, da so ermöglicht wird, große Luftvolumina von Viren zu befreien.

Der Ausbruch der Corona-Pandemie Anfang 2020 zeigt die dringende Notwendigkeit, wirtschaftliche, effiziente und ökologische Verfahren zur Virusinaktivierung vorzuhalten.

Ziel von Weiterentwicklungen ist es, Vorrichtungen bereitzustellen, die es ermöglichen, Medienströme mit einem großen Durchsatz, insbesondere Luftströme, effizient reinigen zu können. Ein weiteres oder alternatives Ziel ist es, auch diejenigen Partikel abzutöten, die vergleichsweise resistent gegen UV-Strahlung sind. Letztlich sind verschiedene Grundstrahlungsdosen bekannt, die in Abhängigkeit der zu tötenden Mikroorganismen voneinander abweichen können.

Die UV-Strahlungsdosis (J/m²) ergibt sich aus der Multiplikation der UV-Bestrahlungsintensität (W/m²) und der UV-Bestrahlungszeit (s).

Letztlich hängt die Inaktivierung der Mikroorganismen insbesondere von der Leistung oder der erreichten Intensität der UV-Strahlung ab. Auch die Distanz des Mediumstromes zur Strahlungsquelle hat einen Einfluss auf die Effizienz der Desinfektion bzw. Reinigung.

Die Strahlungsquelle wird in dem Gehäuse mit einer Halteeinrichtung befestigt, wobei bei einer Mehrzahl von Strahlungsquellen auch eine Mehrzahl von Halteeinrichtungen benötigt werden. Dies führt dazu, dass die Strahlungsquellen vergleichsweise aufwendig - beispielsweise zu Wartungszwecken - montiert und demontiert werden müssen.

Ferner kann keine stabile Fixierung der Strahlungsquellen im Gehäuse erreicht werden, da insbesondere bei an der Außenseite der Strahlungsquelle angreifender Strömung des Mediums ein Verschieben bzw. ein "Wackeln" der Strahlungsquelle(n) nicht sicher verhindert werden kann.

Aufgabe der vorliegenden Erfindung ist es, eine verbesserte Bestrahlungsvorrichtung zur UV-Bestrahlung, insbesondere UV-C-Bestrahlung, bereitzustellen, die insbesondere eine einfache Handhabung und weiter bevorzugt eine effizientere Inaktivierung der Mikroorganismen ermöglicht.

Die erfindungsgemäße Aufgabe wird durch eine Bestrahlungsvorrichtung zur UV-Bestrahlung, insbesondere UV-C-Bestrahlung, eines die Bestrahlungsvorrichtung durchströmenden, vorzugsweise gasförmigen und/oder fluiden, Mediums, insbesondere Wasser oder Luft, gelöst, wobei die Bestrahlungsvorrichtung ein einen Einlass und einen Auslass für das Medium aufweisendes und zu durchströmendes Gehäuse aufweist. Das Gehäuse kann von dem Medium durchströmt werden. Die Bestrahlungsvorrichtung kann insbesondere zur Inaktivierung von in dem Medium befindlichen Mikroorganismen, wie Bakterien, Keime, Schimmel und/oder Viren, verwendet werden. Die Bestrahlungsvorrichtung umfasst weiter wenigstens eine im Inneren des Gehäuses angeordnete und UV-Strahlung, insbesondere UV-C-Strahlung, emittierende Strahlungsquelle zur Bestrahlung des das Gehäuse durchströmenden Mediums.

Das Gehäuse umfasst einen Reflektor, wobei der Reflektor an der der Strahlungsquelle zugewandten Innenseite zumindest bereichsweise, vorzugsweise vollflächig, reflektierend mit einem Reflexionsgrad für die von der Strahlungsquelle emittierte UV-Strahlung von wenigstens 0,6 ausgebildet ist. Insbesondere kann der Reflektor auch durch die Innenseite des Gehäuses gebildet werden. Die Innenseite des Reflektors bzw. des Gehäuses ist insbesondere derart glatt ausgebildet, dass zumindest im Wesentlichen eine direkte bzw. gerichtete Reflektion der eintreffenden Strahlung erfolgen kann.

Erfindungsgemäß weist die Halteeinrichtung eine erste Halteeinheit für die Strahlungsquellen auf. Insbesondere sind alle Strahlungsquellen über die Halteeinheit fixiert und/oder gehalten. Die erste Halteeinheit kann als zusammenhängendes Bauteil ausgebildet sein, wobei sie jedoch aus unterschiedlichen Bestandteilen zusammengesetzt sein kann - also nicht zwingend aus einstückig ausgebildet sein muss, aber einstückig ausgebildet sein kann.

Bei einer ersten erfindungsgemäßen Alternative ist vorgesehen, dass wenigstens zwei Strahlungsquellen derart in der und/oder an der ersten Halteeinheit angeordnet und/oder gelagert sind, dass sich die minimalen Abstände der Strahlungsquellen, insbesondere die minimalen lichten Abstände der Strahlungsquellen, zu der Innenseite des Reflektors voneinander unterscheiden. Somit können zwei Strahlungsquellen an der ersten Halteeinheit angeordnet sein, wobei die Anordnung derart vorgesehen ist, dass unterschiedliche Abstände zu der Innenseite des Reflektors eingehalten werden können.

Bei erfindungsgemäß durchgeführten Versuchen ist festgestellt worden, dass durch eine solche Anordnung vorteilhafte Interferenzeffekte erreicht werden können, die insbesondere zu einer Erhöhung der konstruktiven Interferenz in der Behandlungskammer führen, da vorzugsweise der Anteil an "ausgelöschter" Strahlung - aufgrund von destruktiver Interferenz - reduziert werden kann.

Bei einer zweiten erfindungsgemäßen Alternative, die insbesondere unabhängig von der zuvor genannten ersten erfindungsgemäßen Alternative oder gemeinsam mit der ersten erfindungsgemäßen Alternative realisiert werden kann, ist vorgesehen, dass die erste Halteeinheit über ein erstes Verbindungsmittel der Halteeinrichtung mit dem Gehäuse lösbar verbindbar ist, wobei die erste Halteeinheit wenigstens drei zueinander zumindest bereichsweise beabstandete, als stegförmige und/oder langgestreckte Haltearme ausgebildete erste Haltemittel aufweist. Die ersten Haltemittel sind mit einem Endbereich an einem ersten, mit dem Verbindungsmittel verbundenen Verbindungsbereich gehalten, dabei können die ersten Haltemittel an dem Verbindungsbereich angeordnet, mit diesem verbunden und/oder an diesem gelagert sein. Vorzugsweise sind die Strahlungsquellen, insbesondere jeweils an einem stirnseitigen Endbereich, mit den ersten Haltemitteln, vorzugsweise lösbar und formschlüssig, kraftschlüssig und/oder reibschlüssig, an dem anderen, insbesondere freien, Endbereich der ersten Haltemittel verbunden.

Die ersten Haltemittel können somit insbesondere als Tragarme und/oder Kragarme ausgebildet sein.

Die erfindungsgemäße Ausbildung der ersten Halteeinrichtung gemäß der weiteren erfindungsgemäßen Alternative führt dazu, dass die Handhabung der Strahlungseinheit - als Strahlungseinheit wird nachfolgend das Lampenpaket der Strahlungsquellen bezeichnet - verbessert werden kann. Dies ist insbesondere darauf begründet, dass aufgrund der "gesammelten bzw. gebündelten" Anordnung an der ersten Halteeinheit eine gemeinsame Montage und Demontage der Strahlungseinheit möglich ist und somit insbesondere für Wartungszwecke ein einfacher Austausch der Strahlungsquellen realisiert werden kann.

Zudem ist erfindungsgemäß festgestellt worden, dass über die Anordnung der Strahlungsquellen an wenigstens drei Haltearmen - es sind auch wenigstens drei Strahlungsquellen bevorzugt vorgesehen - erreicht werden kann, dass auch beim Betrieb der Bestrahlungsvorrichtung eine sichere, stabile und zuverlässige Halterung der Strahlungsquellen ermöglicht wird. Die Strahlungsquellen können an den ersten Haltemitteln über Befestigungsmittel oder dergleichen angeordnet sein. Diese Befestigungsmittel können den ersten Haltemitteln zugeordnet sein.

Die Strahlungsquelle ist vorzugsweise derart in dem Gehäuse angeordnet, dass die von der Strahlungsquelle emittierte Strahlung an der Innenseite des Gehäuses, vorzugsweise gerichtet, reflektiert wird und dass die von der Strahlungsquelle emittierte Strahlung mit der, vorzugsweise gerichteten, reflektierten Strahlung konstruktiv interferiert.

In der vorliegenden Anmeldung wird zwischen der gerichteten Reflektion - auch direkte Reflektion genannt - und der diffusen Reflektion unterschieden. Insbesondere ist die Innenseite des Gehäuses bzw. des Reflektors, an der die Strahlung reflektiert wird, derart ausgebildet, dass eine nicht-diffuse, nämlich insbesondere eine gerichtete bzw. direkte, Reflektion der eintreffenden Strahlung erfolgt. Hierfür ist insbesondere eine zumindest im Wesentlichen glatte Oberfläche der Innenseite des Gehäuses erforderlich, die insbesondere gerade nicht rau ausgebildet ist, was andernfalls eine diffuse Reflektion hervorrufen würde.

Ferner wird erfindungsgemäß die Strahlungsquelle insbesondere so angeordnet, dass die konstruktive Interferenz gezielt zur Erhöhung der Effizienz der Bestrahlungsvorrichtung eingesetzt wird. In diesem Zusammenhang sind unterschiedliche Ausführungen der erfindungsgemäßen Bestrahlungsvorrichtung denkbar, so dass ein auf konstruktiver Interferenz beruhendes Interferenzbild bzw. Interferenzmuster erreicht werden kann. Insbesondere kann entweder die Gehäusewand, insbesondere eine die Innenseite aufweisende Innenwand des Gehäuses, entsprechend ausgebildet sein und/oder die Strahlungsquelle kann relativ zu der Gehäuseinnenwand in einer bestimmten Art angeordnet sein.

Das Innere des Gehäuses bzw. der umschlossene Innenraum des Gehäuses kann insbesondere als UV-Behandlungskammer zur Behandlung des Mediums angesehen werden.

Bevorzugt ist die Innenwandung ein bzw. der Reflektor, der weiter bevorzugt als in das Gehäuse eingeschobene und/oder austauschbare Gehäusekomponente ausgebildet ist.

Unter dem Reflexionsgrad wird im Sinne der Erfindung das Verhältnis zwischen reflektierter und einfallender Intensität als Energiegröße verstanden. Der Reflexionsgrad kann insbesondere in Abhängigkeit des Materials der Innenwandung, auf die die Strahlung auftrifft, und von der Strahlung selbst abhängen.

Interferenz beschreibt dabei die Änderung der Amplitude von zwei oder mehr Wellen nach dem Superpositionsprinzip. Interferenz tritt grundsätzlich bei unterschiedlichen Arten von Wellen auf, wobei im Rahmen der Erfindung die UV-Strahlung für die Interferenz relevant ist.

Destruktive Interferenz bezeichnet ein Phänomen, bei dem an einen bestimmten Ort die Wellen sich gegenseitig auslöschen. Dahingegen kennzeichnet die konstruktive Interferenz diejenigen Orte, wo sich die Wellen - sofern sie aufeinander treffen - verstärken. Die hat eine Erhöhung der Amplitude zur Folge.

Erfindungsgemäß wird insbesondere darauf abgezielt, die Amplitude der, insbesondere maximalen, Gesamt-Intensität der wechselwirkenden UV-Strahlung durch konstruktive Interferenz zu erhöhen. Im Inneren des Gehäuses entsteht somit ein durch konstruktive Interferenz geprägtes Interferenzmuster. In denjenigen Bereichen innerhalb des Gehäuses, wo die konstruktive Interferenz vorherrscht, kann eine besonders effiziente Abtötung der Viren ermöglicht werden.

Bei einer weiteren ganz besonders bevorzugten Ausführungsform des Erfindungsgedankens ist vorgesehen, dass die Innenseite des Gehäuses und/oder die Innenseite des Reflektors ein Reflexionsgrad für die von der Strahlungsquelle emittierte UV-Strahlung von wenigstens 0,7, bevorzugt von wenigstens 0,8, weiter bevorzugt von wenigstens 0,9, aufweist. Insbesondere ist der Reflexionsgrad derart gewählt, dass die erfindungsgemäßen Interferenzen hervorgerufen werden können, die durch Wechselwirkung der an der Innenseite des Gehäuses reflektierten, vorzugsweise gerichteten, Strahlung mit der im Inneren des Gehäuses befindlichen Strahlung hervorgerufen werden können.

Darüber hinaus ist bei einer weiteren bevorzugten Ausführungsform vorgesehen, dass die Strahlungsquelle lösbar mit der Halteeinrichtung verbunden ist. Bevorzugt ist jede Strahlungsquelle lösbar mit der Halteeinrichtung verbunden. Eine lösbare Anordnung ermöglicht den Vorteil, dass eine Strahlungsquelle, die beschädigt ist oder ausgetauscht werden muss, aus der Halteeinrichtung entnommen werden kann. In diesem Zusammenhang kann vorgesehen sein, dass die Strahlungsquelle gemeinsam mit der Halteeinrichtung zunächst aus dem Reflektor entfernt wird, wobei anschließend ein Austausch der Strahlungsquelle erfolgt.

Bei einer besonders bevorzugten Ausführungsform des Erfindungsgedankens ist vorgesehen, dass wenigstens zwei Strahlungsquellen derart in dem und/oder an der ersten Halteeinheit angeordnet und/oder gelagert sind, dass die entlang der jeweiligen Längsrichtung der wenigstens zwei Strahlungsquellen verlaufenden Mittelachsen miteinander einen Winkel einschließen. Alternativ oder zusätzlich bevorzugt vorgesehen, dass wenigstens zwei Strahlungsquellen derart in dem und/oder an der ersten Halteeinheit angeordnet bzw. gelagert sind, dass die entlang der jeweiligen Längsrichtung der wenigstens zwei Strahlungsquellen verlaufenden Mittelachsen miteinander einen Winkel zwischen 1° bis 160°, bevorzugt zwischen 10° bis 40°, einschließen.

Erfindungsgemäß wird unter der Mittelachse insbesondere die Längsachse des Reflektors bzw. des Gehäuses oder der Strahlungsquelle verstanden. Die Mittelachse liegt bzw. verläuft insbesondere in der jeweiligen Mitte des Körpers und/oder im Schwerpunkt des jeweiligen Körpers und bildet vorzugsweise die Symmetrieachse. Sofern der Körper nicht symmetrisch ausgebildet ist, bildet die Mittelachse des jeweiligen Körpers - das heißt des Reflektors, des Gehäuses und/oder der Strahlungsquelle - die annähernde Symmetrieachse des Körpers. Somit werden erfindungsgemäß auch Mittelachsen von solchen Körpern umfasst, die nicht symmetrisch sind.

Insbesondere verläuft die Mittelachse der Strahlungsquelle oder des Reflektors bzw. des Gehäuses durch den Schwerpunkt und/oder den Mittelpunkt der Strahlungsquelle bzw. des Gehäuses. Die Mittelachse verläuft vorzugsweise in Längserstreckung des Reflektors bzw. des Gehäuses oder der Strahlungsquelle, wobei die Strahlungsquelle oder der Reflektor bzw. das Gehäuse langgestreckt ausgebildet sind.

Eine Längserstreckung ist insbesondere derart zu verstehen, dass die Länge des Körpers die Breite des Körpers übersteigt.

Demnach können die Strahlungsquellen insbesondere miteinander verdreht und/oder zueinander tordiert angeordnet sein. Beim Zustandekommen der Erfindung ist festgestellt worden, dass eine solche Anordnung ermöglicht, eine Erhöhung der Bestrahlungsintensität zu erreichen. Diese Erhöhung wird letztlich insbesondere dadurch gewährleistet, dass der Anteil an ausgelöschter Strahlung (destruktive Interferenz) in der Behandlungskammer reduziert werden kann. Der Verlauf der von den Strahlungsquellen ausgesendeten Strahlung, die wiederrum mit der, vorzugsweise gerichtet, reflektierten Strahlung wechselwirkt, kann somit in vorteilhafter Weise beeinflusst werden. Die exakte Ausrichtung der Verdrehung der Strahlungsquellen kann in Abhängigkeit des gewählten Reflektors und der Anzahl der Strahlungsquellen bevorzugt experimentell ermittelt werden. Letztlich ist das entstehende Gesamt-Interferenzbild nur schwer simulierbar, so dass zur bestmöglichen Anordnung insbesondere eine experimentelle Bestimmung durchführbar ist.

Vorzugsweise sind wenigstens zwei Strahlungsquellen derart in dem und/oder an der ersten Halteeinheit angeordnet bzw. gelagert sind, dass die minimalen (lichten) Abstände zu der Innenseite des Reflektors sich voneinander um wenigstens 5%, bevorzugt wenigstens 20%, weiter bevorzugt wenigstens 30%, unterscheiden. Durch diese Anordnung der Strahlungsquellen kann insbesondere ein verbessertes Strahlungsbild erreicht werden, was wiederum zu einer Verbesserung der zu verabreichenden UV-Strahlungsdosis führen kann. Ferner kann durch die unterschiedlichen Abstände auch ermöglicht werden, dass die Strahlungsquellen als gemeinsames Lampenpaket handhabbar sind, wodurch die gesamte Handhabung der Bestrahlungsvorrichtung vereinfacht werden kann.

Wie zuvor erläutert, kann das erste Haltemittel langgestreckt ausgebildet sein. Alternativ oder zusätzlich kann vorgesehen sein, dass wenigstens zwei erste Haltemittel eine sich voneinander unterscheidende Länge aufweisen. Alternativ oder zusätzlich kann vorgesehen sein, dass das erste Haltemittel wenigstens zwei Anordnungsbereiche, vorzugsweise zur Anordnung der Befestigungsmittel, zur Verbindung mit der Strahlungsquelle aufweist.

Über die Anordnungsbereiche kann beispielsweise eine versetzte Anordnung der Strahlungsquellen in Bezug zueinander erreicht werden. So kann beispielsweise eine Strahlungsquelle an dem einen ersten Haltemittel weiter außen (in Bezug zum freien Endbereich des ersten Haltemittels) angeordnet sein als eine unmittelbar benachbarte Strahlungsquelle, die an einem weiteren ersten Haltemittel angeordnet ist, jedoch an einem gegenüber dem zuvor angesprochenen Anordnungsbereich versetzt angeordneten Anordnungsbereich.

Vorzugsweise ist die erste Halteeinheit derart ausgebildet, dass wenigstens zwei zwischen zwei unmittelbar benachbarten ersten Haltemitteln eingeschlossene Winkel zueinander unterschiedlich ausgebildet sind und insbesondere um wenigstens 5 %, bevorzugt wenigstens 10 %, zueinander abweichen bzw. sich voneinander unterscheiden. Besonders bevorzugt sind alle ersten Haltemittel zueinander derart angeordnet, dass alle eingeschlossenen Winkel zwischen unmittelbar benachbarten ersten Haltemitteln zueinander unterschiedlich sind. Dies kann zur weiteren vorteilhaften Wirkung beitragen, dass durch die schräge Anordnung der Strahlungsquellen in dem Reflektor konstruktive Interferenzeffekte erreicht werden können. Durch die unterschiedliche Anordnung der ersten Haltemittel, bevorzugt an dem ersten Verbindungsbereich, kann dieser Effekt nun weiter verstärkt werden.

Die Halteeinrichtung kann derart ausgebildet sein, dass die Mittelachse von wenigstens einer Strahlungsquelle einen Winkel zur Mittelachse des Reflektors einschließt. Alternativ oder zusätzlich kann vorgesehen sein, dass alle Mittelachsen der Strahlungsquellen jeweils einen Winkel zur Mittelachse des Reflektors einschließen.

Erfindungsgemäß ist festgestellt worden, dass durch die vorgenannte schräge Anordnung zwischen der Mittelachse der wenigstens einen Strahlungsquelle und der Mittelachse des Reflektors bzw. des Gehäuses eine Erhöhung der konstruktiven Interferenz und somit insbesondere eine Verbesserung der zu verabreichenden UV-Strahlungsdosis, mit der das Medium behandelt wird, erreicht werden kann. Dass sich eine solche Verbesserung durch eine Schrägstellung der Strahlungsquelle einstellt, ist für den Fachmann nicht zu erwarten gewesen.

Letztlich ist erfindungsgemäß festgestellt worden, dass insbesondere gezielt die Interferenz zwischen der an der Innenseite des Reflektors direkt reflektierten Strahlung und der von der Strahlungsquelle emittierten Strahlung gesteuert werden kann, insbesondere derart, dass sich eine Erhöhung der der Amplitude der Strahlungsintensität im Vergleich zu einer "geraden" Anordnung ergibt.

Die Strahlung wird zudem insbesondere mehrfach an der Innenseite des Reflektors direkt reflektiert, so dass sich durch die schräge Anordnung - auf nicht vorhersehbare Weise - eine Erhöhung der Intensität der Strahlung ergibt, was zu einer verbesserten UV-Dosis führt. Die Ausbildung des entstehenden Interferenzbildes ist derart komplex, dass das entstehende Gesamt-Interferenzbild ohne experimentelle Versuche, wie sie erfindungsgemäß beim Zustandekommen der Erfindung durchgeführt worden sind, nicht sicher vorhergesagt und/oder simuliert werden kann - dies ist insbesondere auf die Inter-Reflexionen begründet, die im Reflektor auftreten.

Schließlich wird erfindungsgemäß vermieden, dass die von der Strahlungsquelle emittierte Strahlung aufgrund der Reflektion an der Innenseite des Reflektors größtenteils ausgelöscht wird. Die diesbezüglichen "Auslöschungseffekte" werden im Stand der Technik bei der im Reflektor vorhandenen geraden Ausrichtung der Strahlungsquelle "in Kauf genommen", da letztlich im Stand der Technik nicht bekannt gewesen ist, wie solche Auslöschungseffekte vermieden werden können. Dass es überhaupt möglich ist, die konstruktive Interferenz zu erhöhen, ist dem Fachmann in der Praxis bisher nicht bekannt gewesen.

Vorzugsweise kann die biologische Dosis, die insbesondere auf Basis des View-Factors ermittelt wird, deutlich erhöht werden, vorzugsweise um wenigstens 5 %, weiter bevorzugt um wenigstens 10 %. Eine solche Erhöhung wird dadurch erreicht, dass, wie zuvor erläutert, der Verlust in den verschiedenen Reflexionsstufen der von der Strahlungsquelle emittierten Strahlung deutlich herabgesenkt werden kann, nämlich aufgrund der vorteilhaften Positionierung der Strahlungsquelle im Reflektor.

Die schräge Anordnung der Strahlungsquelle im Reflektor ermöglicht es somit, das insgesamt erreichte Abtötungsergebnis der in dem Medium befindlichen Mikroorganismen zu erhöhen und somit eine verbesserte Desinfektion des Mediums bereitzustellen.

Bei einer besonders bevorzugten Ausführungsform ist vorgesehen, dass der eingeschlossene Winkel zwischen der Mittelachse der wenigstens einen Strahlungsquelle und der Mittelachse des Reflektors zwischen arcsin((0,2 • D) / L) und arcsin((4 • D) / L) liegt. Somit kann insbesondere der Abstand zwischen dem einen stirnseitigen Ende der Strahlungsquelle und dem anderen stirnseitigen Ende bzw. die erzeugte Maximalverschiebung zwischen 0,2 • D und 4 • D liegen. In diesem Zusammenhang gibt D den, insbesondere maximalen, Durchmesser der Strahlungsquelle an, wobei L die Länge der Strahlungsquelle referenziert. Insgesamt wird also ein Schrägversatz über die gesamte Länge der jeweiligen Strahlungsquelle zwischen 0,2 • D bis 4 • D erreicht.

In diesem Zusammenhang versteht es sich, dass die Strahlungsquellen (zueinander) die gleiche oder auch eine unterschiedliche Länge oder Durchmesser aufweisen können. Das vorgenannte Verhältnis des Winkels bezieht sich dabei auf die jeweilige Länge und den jeweiligen Durchmesser der betrachteten Strahlungsquelle. Sofern der Durchmesser der Strahlungsquelle variiert, bezeichnet D insbesondere den maximalen und/oder den mittleren Durchmesser.

Besonders bevorzugt liegt der eingeschlossene Winkel zwischen der Mittelachse der wenigstens einen Strahlungsquelle und der Mittelachse des Reflektors zwischen arcsin((0,5 • D) / L) und arcsin((3 • D) / L), weiter bevorzugt zwischen arcsin(D / L) und arcsin((2 • D) / L). Alternativ oder zusätzlich kann vorgesehen sein, dass der eingeschlossene Winkel zwischen der Mittelachse der wenigstens einen Strahlungsquelle und der Mittelachse des Reflektors zwischen 0,5° bis 15°, weiter bevorzugt zwischen 2° bis 10°, vorzugsweise zwischen 2° +/- 0,5°, liegt.

Bei einer weiteren bevorzugten Ausführungsform ist vorgesehen, dass wenigstens ein erstes Haltemittel, bevorzugt wenigstens zwei erste Haltemittel, vorzugsweise alle ersten Haltemittel, über ein mit dem Verbindungsbereich verbundenes erstes Verstellmittel verstellbar ist. Das erste Verstellmittel kann beispielsweise eine Schraubverbindung, vorzugsweise in Kombination mit einem Langloch, und/oder eine teleskopierbare Verstellmöglichkeit oder dergleichen aufweisen.

Letztlich kann das erste Verstellmittel derart ausgebildet sein, dass die Schrägstellung der Mittelachse der an dem ersten Haltemittel befestigten Strahlungsquelle in Bezug zu der Mittelachse des Reflektors verstellbar ist. In diesem Zusammenhang versteht es sich, dass das erste Verstellmittel nur bedarfsweise "aktiviert" bzw. gelöst wird. So kann beispielsweise das erste Verstellmittel im gelösten Zustand eine Verstellung der Schrägstellung der Mittelachse der jeweiligen an dem ersten Haltemittel befestigten Strahlungsquelle ermöglichen, wobei nach erfolgter Ausrichtung eine erneute Feststellung bzw. Sicherung des ersten Verstellmittels erfolgen kann - beispielsweise durch ein Anziehen der Schraubverbindung. Dadurch kann im festgestellten Zustand des ersten Verstellmittels die Strahlungsquelle fest an dem ersten Haltemittel angeordnet sein, insbesondere so dass keine erneute Verstellung der Schrägstellung der Mittelachse der Strahlungsquelle möglich ist.

Ferner ist bei einer weiteren bevorzugten Ausführungsform vorgesehen, dass die Strahlungsquelle zumindest im Wesentlichen stabförmig ausgebildet ist. Alternativ oder zusätzlich kann vorgesehen sein, dass die Strahlungsquelle zumindest im Wesentlichen zylinderförmig und/oder langgestreckt ausgebildet ist. Insbesondere verläuft die Längserstreckung der Strahlungsquelle zumindest im Wesentlichen - das heißt unter Berücksichtigung der schrägen und/oder winkligen Anordnung der Strahlungsquelle im Reflektor - in Längsrichtung des Gehäuses bzw. des Reflektors. Demgemäß verläuft die Längserstreckung der Strahlungsquelle insbesondere nicht orthogonal zur Längserstreckung des Gehäuses und/oder des Reflektors.

Bei einer weiteren bevorzugten Ausführungsform sind die Mittelachsen, insbesondere wenigstens zwei Mittelachsen, weiter bevorzugt wenigstens vier Mittelachsen, insbesondere alle Mittelachsen, der Strahlungsquellen zueinander parallel angeordnet.

Alternativ oder zusätzlich kann vorgesehen sein, dass wenigstens zwei Mittelachsen, bevorzugt wenigstens drei Mittelachsen, weiter bevorzugt wenigstens vier Mittelachsen, der Strahlungsquellen jeweils zueinander versetzt, vorzugsweise schräg, angeordnet sind. Demnach können die Strahlungsquellen auch miteinander verdreht, tordiert und/oder verdrillt angeordnet sein. Insbesondere kann der eingeschlossene Winkel zwischen zwei benachbarten Strahlungsquellen, insbesondere zwischen den benachbarten Mittelachsen der benachbarten Strahlungsquellen, zwischen 1° bis 120°, weiter bevorzugt zwischen 5° bis 90°, weiter bevorzugt zwischen 10° bis 40°, betragen. Der vorgenannte Winkel gibt insbesondere den Grad der Verdrillung zwischen den Strahlungsquellen an.

Beim Zustandekommen der Erfindung hat sich überraschenderweise gezeigt, dass durch eine Kombination der Schrägstellung der Strahlungsquelle im Reflektor und der zusätzlichen Tordierung und/oder Verdrillung der Strahlungsquellen zueinander ein solches Interferenzbild - erzeugt durch die an dem Reflektor, vorzugsweise gerichtete, reflektierte Strahlung und die von der Strahlungsquelle ausgesendete Strahlung sowie die mehrfachen Reflektionen an der Innenseite des Reflektors - ermöglicht wird, das zu einer signifikanten Erhöhung der maximalen Intensität der in dem Reflektor befindlichen Strahlung führt. Letztlich führt die Schrägstellung der Strahlungsquellen - sowohl zueinander als auch zu der Mittelachse des Reflektors - dazu, dass eine Auslöschung der Maxima (aufgrund von destruktiver Interferenz) der von der Strahlungsquelle ausgesendeten Strahlung verhindert bzw. deutlich reduziert werden kann, so dass die verstärkenden Effekte der Überlagerung der Strahlung (nämlich die konstruktive Interferenz) genutzt werden können. Warum sich genau eine solche Interferenzwirkung bzw. ein solches Interferenzbild ergibt, ist abschließend nicht sicher feststellbar. Das entstehende Interferenzbild hängt von einer Vielzahl von Faktoren ab, wobei das erzeugte Strahlungsbild ebenfalls nicht hinreichend genau in Modellen abbildbar ist. Deshalb ist es nicht zu erwarten gewesen, dass sich eine signifikante Verbesserung der zu verabreichenden Strahlungsdosis und/oder der erreichten maximalen Intensität, insbesondere um wenigstens 20 %, im Vergleich zum Stand der Technik ergibt. Allerdings ist erfindungsgemäß eine derartige Verbesserung durch die Schrägstellung der Strahlungsquelle(n) sichergestellt worden.

Besonders bevorzugt sind die Mittelachsen der Strahlungsquellen zueinander schräg und/oder windschief angeordnet.

Zudem kann vorgesehen sein, dass eine Mehrzahl von Strahlungsquellen an der Halteeinrichtung gehalten und/oder fixiert ist. Insbesondere schließt jede Mittelachse jeder Strahlungsquelle einen Winkel, bevorzugt in der vorgenannten Größenordnung, zu der Mittelachse des Reflektors ein.

Die vorgenannte Schrägstellung ermöglicht eine Verbesserung der konstruktiven Interferenz und somit des gesamten Strahlungsbildes bzw. Interferenzbildes im Reflektor. Beim Zustandekommen der Erfindung ist überraschenderweise festgestellt worden, dass durch einen Schrägversatz, der insbesondere von dem Durchmesser und der Länge der jeweiligen Strahlungsquelle abhängt, eine Erhöhung der maximalen Strahlungsintensität erreicht werden kann.

Darüber hinaus ist bei einer weiteren bevorzugten Ausführungsform vorgesehen, dass die Strahlungsquellen zueinander gleich beabstandet sind. Insbesondere erstreckt sich die Gleich-Beabstandung der Strahlungsquellen über die gesamte Länge der jeweiligen Strahlungsquellen. Alternativ oder zusätzlich kann vorgesehen sein, dass, wie zuvor erläutert, wenigstens zwei, insbesondere wenigstens zwei unmittelbar benachbarte, Strahlungsquellen mit ihrer Mittelachse einen unterschiedlichen Winkel zu der Mittelachse des Reflektors einschließen. Demnach können die Strahlungsquellen auch zueinander tordiert und/oder verdrillt angeordnet sein, was erfindungsgemäß die zuvor angesprochenen vorteilhaften Effekte der Erhöhung der konstruktiven Interferenz zur Folge hat.

Eine parallele Beabstandung der Strahlungsquellen ist mit dem Vorteil verbunden, dass eine einfache Handhabung des "Lampenpaketes" - das heißt der an der Halteeinrichtung befestigten Strahlungsquellen - erfolgen kann sowie ein einfaches Austauschen der einzelnen Strahlungsquellen, da nicht auf eine etwaige Verdrillung zwischen den Strahlungsquellen geachtet werden muss.

Ferner kann die erste Halteeinheit zur Energiezuführung zu den Strahlungsquellen ausgebildet sein. Dabei können insbesondere in dem ersten Verbindungsmittel, in der ersten Halteeinheit und/oder in den ersten Haltemitteln Energieversorgungsleitungen, insbesondere Stromleitungen, Netzanschlussleitungen und dergleichen, angeordnet sein. Die Energieversorgungsleitungen können derart ausgebildet sein, dass die korrekte Funktionsweise der Strahlungsquellen, die vorzugsweise als LED ausgebildet sind, sichergestellt werden kann. Die zum Betrieb der Strahlungsquellen benötigte Energie kann somit den Strahlungsquellen über das erste Haltemittel und/oder über die erste Halteeinheit zugeführt werden. Somit können die im Inneren des Reflektors angeordneten Strahlungsquellen elektrisch bzw. energetisch angebunden werden.

In diesem Zusammenhang versteht es sich, dass etwaig vorgesehene Vorschaltgeräte, Netzanschlussteile und dergleichen außerhalb des Reflektors, insbesondere an der Außenseite des Gehäuses, angeordnet werden können und über Energieversorgungsleitungen, die durch die erste Halteeinheit geführt werden können, mit den Strahlungsquellen verbunden werden können. Demnach kann durch die vorteilhafte Leitungsführung vermieden werden, dass Energieversorgungsleitungen im Innenraum des Reflektors, insbesondere unbefestigt, mögliche destruktive Interferenz fördern könnten oder bei der Montage oder Demontage der Strahlungseinheit bzw. des Lampenpaketes stören oder gar zur Beschädigung des Lampenpaketes führen könnten.

Bei einer weiteren bevorzugten Ausführungsform ist vorgesehen, dass eine, vorzugsweise komplementär und/oder korrespondierend zu der ersten Halteeinheit ausgebildete, zweite Halteeinheit der Halteeinrichtung vorgesehen ist. Die zweite Halteeinheit kann zur Halterung bzw. Fixierung der Strahlungsquellen ausgebildet sein. Insbesondere sind die Strahlungsquellen mit dem weiteren stirnseitigen Endbereich mit der zweiten Halteeinheit zumindest mittelbar verbunden. Die zweite Halteeinheit kann über wenigstens ein zweites Verbindungsmittel der Halteeinrichtung mit dem Gehäuse, vorzugsweise lösbar, verbindbar sein. Vorzugsweise kann die zweite Halteeinheit eine Mehrzahl von zueinander zumindest bereichsweise beabstandeten zweiten Haltemitteln, vorzugsweise ausgebildet als, insbesondere stegförmige, Haltearme, aufweisen.

In diesem Zusammenhang versteht es sich, dass die zweiten Haltearme bzw. die zweiten Haltemittel der zweiten Halteeinheit zueinander unterschiedlich ausgebildet sein können. An den zweiten Haltemitteln können die Strahlungsquellen derart befestigt werden, dass jeweils ein zweites Haltemittel einer Strahlungsquelle zugeordnet ist und zur Befestigung einer Strahlungsquelle dient. Alternativ oder zusätzlich kann vorgesehen sein, dass jedem ersten Haltemittel ein korrespondierendes zweites Haltemittel zugordnet ist.

Eine Beabstandung der Strahlungsquellen zueinander kann somit über eine zumindest bereichsweise vorgesehene Beabstandung der zweiten Haltemittel erreicht werden. Dadurch, dass die zweiten Haltemittel der zweiten Halteeinheit zugeordnet und alle zweiten Haltemittel miteinander zumindest mittelbar verbunden sind, kann eine kompakte Ausbildung der gesamten Strahlungseinheit - d.h. die die Strahlungsquellen aufweisende Strahlungseinheit - erreicht werden. Die zweite Halteeinheit ermöglicht es daher gemeinsam mit der ersten Halteeinheit, die Strahlungseinheit kompakt dem Gehäuse und/oder dem Reflektor zu entnehmen und so letztlich eine einfache Montage und Demontage der Strahlungseinheit - beispielsweise zu Wartungszwecken - aus dem Gehäuse zu ermöglichen.

Durch die Beabstandung der zweiten Haltemittel kann insbesondere eine zumindest im Wesentlichen sonnenförmige und/oder sternförmige Ausbildung der zweiten Halteeinheit erreicht werden, wobei die zweiten Haltemittel ausgehend von einem gemeinsamen Ausgangspunkt abstehen.

Ferner können die zweiten Haltemittel mit einem zweiten Verbindungsbereich der zweiten Halteeinheit verbunden sein. Der zweite Verbindungsbereich kann mit dem zweiten Verbindungsmittel, vorzugsweise unmittelbar, verbunden sein und/oder einstückig mit diesem ausgebildet sein.

Letztlich können die zweiten Haltemittel über den zweiten Verbindungsbereich mit dem zweiten Verbindungsmittel und somit lösbar mit dem Gehäuse und/oder dem Reflektor verbunden werden. Die zweiten Haltemittel können mit jeweils einem Endbereich mit dem zweiten Verbindungsbereich verbunden oder an diesem gelagert sein. Der weitere Endbereich des, vorzugsweise langgestreckten, zweiten Haltemittels kann frei angeordnet sein - das heißt nicht mit einem weiteren Bestandteil der zweiten Halteeinheit unmittelbar verbunden sein.

Ferner kann somit das zweite Haltemittel als Tragarm und/oder Kragarm ausgebildet sein, der an einem Endbereich an dem zweiten Verbindungsbereich angeordnet und/oder gelagert ist. Der zweite Verbindungsbereich kann somit das Zentrum der zweiten Halteeinheit bilden. Der zweite Verbindungsbereich muss allerdings nicht im Schwerpunkt der zweiten Halteeinheit angeordnet sein, sondern kann auch außerhalb des Schwerpunktes der zweiten Halteeinheit angeordnet sein.

Zudem kann die Strahlungsquelle, vorzugsweise an einem weiteren stirnseitigen Endbereich, mit dem zweiten Haltemittel verbunden sein. Dabei kann die Strahlungsquelle insbesondere zwei stirnseitige Endbereiche aufweisen, die jeweils mit dem ersten bzw. zweiten Haltemittel verbunden sind.

Die Verbindung zu dem zweiten Haltemittel kann insbesondere zum einen lösbar und zum anderen formschlüssig, kraftschlüssig und/oder reibschlüssig ausgebildet sein. In diesem Zusammenhang versteht es sich, dass nicht zwingend die weitere Stirnseite der Strahlungsquelle mit dem zweiten Haltemittel verbunden sein muss, sondern der weitere stirnseitige Endbereich umfasst letztlich den Bereich der Strahlungsquelle, der die weitere Stirnseite einschließt.

Das zweite Haltemittel kann wie das erste Haltemittel außerdem zur Befestigung mit der Strahlungsquelle wenigstens ein Befestigungsmittel, beispielsweise wenigstens einen Clip, wenigstens eine Klammer, wenigstens einen Federschenkel oder dergleichen aufweisen, das bzw. die zur lösbaren Anordnung der Strahlungsquelle ausgebildet sind/ist. Dieses Befestigungsmittel kann darüber hinaus vorzugsweise an dem zweiten Haltemittel verschiebbar angeordnet sein, insbesondere so dass eine Justage der Anordnung der Strahlungsquellen erfolgen kann. Alternativ oder zusätzlich kann vorgesehen sein, dass das Befestigungsmittel einstückig mit den weiteren Bestandteilen des zweiten Haltemittels ausgebildet ist. In diesem Zusammenhang versteht es sich, dass die Befestigungsmittel als Bestandteil des zweiten Haltemittels angesehen werden.

Zudem kann erfindungsgemäß die Strahlungsquelle durch unterschiedliche Befestigungsarten mit dem zweiten Haltemittel verbunden sein. Bevorzugt sind die Befestigungsmittel der zweiten Haltemittel zumindest im Wesentlichen baugleich zu den Befestigungsmitteln des ersten Haltemittels ausgebildet.

Des Weiteren kann vorgesehen sein, dass die Strahlungsquelle in dem zweiten Haltemittel zumindest bereichsweise aufgenommen ist, insbesondere so dass die Stirnseite gegenüber dem zweiten Haltemittel absteht. Zur Aufnahme der Strahlungsquelle kann das zweite Haltemittel ebenfalls entsprechende Befestigungsmittel, beispielsweise Spannklemmen, aufweisen.

Außerdem ist bei einer weiteren bevorzugten Ausführungsform vorgesehen, dass das zweite Haltemittel, vorzugsweise alle zweiten Haltemittel, mit dem einen Endbereich mit dem zweiten Verbindungsbereich verbunden ist. Alternativ oder zusätzlich kann vorgesehen sein, dass das zweite Haltemittel an seinem freien Endbereich - insbesondere dem nicht-gelagerten Endbereich - mit der Strahlungsquelle verbunden ist. Auch in diesem Zusammenhang versteht es sich, dass der Endbereich des zweiten Haltemittels nicht zwingend das äußerste Ende des zweiten Haltemittels referenzieren muss, sondern der Endbereich kann das äußerste Ende und einen sich daran anschließenden Bereich umfassen. Der Endbereich des zweiten Haltemittels erstreckt sich dabei über höchstens 30 %, vorzugsweise höchstens 20 %, der Länge des langgestreckten zweiten Haltemittels.

Bei einer weiteren bevorzugten Ausführungsform ist vorgesehen, dass wenigstens ein zweites Haltemittel, bevorzugt wenigstens zwei zweite Haltemittel, vorzugsweise alle zweiten Haltemittel, über jeweils ein mit dem zweiten Verbindungsbereich verbundenes zweites Verstellmittel verstellbar ist. Das zweite Verstellmittel kann beispielsweise eine Schraubverbindung, vorzugsweise in Kombination mit einem Langloch, und/oder eine teleskopierbare Verstellmöglichkeit oder dergleichen aufweisen.

Letztlich kann das zweite Verstellmittel derart ausgebildet sein, dass die Schrägstellung der Mittelachse der an dem ersten Haltemittel befestigten Strahlungsquelle in Bezug zu der Mittelachse des Reflektors verstellbar ist. In diesem Zusammenhang versteht es sich, dass das zweite Verstellmittel nur bedarfsweise "aktiviert" bzw. gelöst wird. So kann beispielsweise das zweite Verstellmittel im gelösten Zustand eine Verstellung der Schrägstellung der Mittelachse der jeweiligen an dem zweiten Haltemittel befestigten Strahlungsquelle ermöglichen, wobei nach erfolgter Ausrichtung eine erneute Feststellung bzw. Sicherung des zweiten Verstellmittels erfolgen kann - beispielsweise durch ein Anziehen der Schraubverbindung. Dadurch kann im festgestellten Zustand des zweiten Verstellmittels die Strahlungsquelle fest an dem zweiten Haltemittel angeordnet sein, insbesondere so dass keine erneute Verstellung der Schrägstellung der Mittelachse der Strahlungsquelle gegeben ist.

Wie zuvor erläutert, kann das zweite Haltemittel langgestreckt ausgebildet sein. Alternativ oder zusätzlich kann vorgesehen sein, dass wenigstens zwei zweite Haltemittel eine sich voneinander unterscheidende Länge aufweisen. Alternativ oder zusätzlich kann vorgesehen sein, dass das zweite Haltemittel wenigstens zwei zweite Anordnungsbereiche, vorzugsweise zur Anordnung der Befestigungsmittel, zur Verbindung mit der Strahlungsquelle aufweist.

Vorzugsweise ist die zweite Halteeinheit derart ausgebildet, dass wenigstens zwei zwischen zwei unmittelbar benachbarten zweiten Haltemitteln eingeschlossene Winkel zueinander unterschiedlich ausgebildet sind und insbesondere um wenigstens 5 %, bevorzugt wenigstens 10 %, zueinander abweichen bzw. sich voneinander unterscheiden. Besonders bevorzugt sind alle zweiten Haltemittel zueinander derart angeordnet, dass alle eingeschlossenen Winkel zwischen unmittelbar benachbarten zweiten Haltemitteln zueinander unterschiedlich sind. Dies kann zur weiteren vorteilhaften Wirkung beitragen, dass durch die schräge Anordnung der Strahlungsquellen in dem Reflektor konstruktive Interferenzeffekte erreicht werden können. Durch die unterschiedliche Anordnung der zweiten Haltemittel, bevorzugt an dem zweiten Verbindungsbereich, kann dieser Effekt nun weiter verstärkt werden.

Vorzugsweise ist die erste Halteeinheit, insbesondere der Verbindungsbereich der ersten Halteeinheit, mit der zweiten Halteeinheit, insbesondere mit dem zweiten Verbindungsbereich, über ein langgestrecktes Verbindungsteil verbunden. Das Verbindungsteil kann insbesondere steif und/oder stabil ausgebildet sein. Bevorzugt ist das Verbindungsteil an seiner dem Reflektor zugewandten Außenseite reflektierend, vorzugsweise mit einem Reflexionsgrad von wenigstens 0,6, bevorzugt von wenigstens 0,8, ausgebildet.

Das Verbindungsteil kann die Stabilität der gesamten Strahlungseinheit bzw. des Lampenpaketes gewährleisten. So ermöglicht das Verbindungsteil, dass die erste Halteeinheit und die zweite Halteeinheit nicht nur über die Strahlungsquellen miteinander verbunden sind, sondern auch über das Verbindungsteil. Das Verbindungsteil kann grundsätzlich unterschiedlich ausgebildet sein, beispielsweise zylinderförmig, rohrförmig oder dergleichen.

Insbesondere kann das Verbindungsteil mittig zwischen den Strahlungsquellen angeordnet und/oder von den Strahlungsquellen umgeben sein. Somit kann das Verbindungsteil im mittleren Bereich bzw. im Zentrum der Strahlungseinheit bzw. des Lampenpaketes angeordnet sein.

Weiter insbesondere ist das Verbindungsteil nicht unmittelbar mit den ersten und/oder zweiten Haltemitteln und/oder mit den jeweiligen Strahlungsquellen verbunden. Vorzugsweise ist das Verbindungsteil an den Verbindungsbereichen der ersten und zweiten Halteeinheit angeordnet. Das Verbindungsteil ermöglicht somit ebenfalls eine einfache Montage und Demontage des gesamten Lampenpaketes und darüber hinaus auch eine Fixierung der Schrägstellung der Strahlungsquellen, insbesondere bei Durchströmung des Reflektors. So kann beispielsweise vermieden werden, dass die Strahlungsquellen im Hinblick auf ihre Ausrichtung im Verhältnis zur Mittelachse des Reflektors "wackeln" - und zwar aufgrund der an der Strahlungsquelle angreifenden Strömung des Mediums. Somit trägt das Verbindungsteil nicht nur zur Stabilität, sondern auch zur verbesserten Funktionsweise der gesamten Bestrahlungsvorrichtung bei.

Besonders bevorzugt ist, dass die zweite Halteinheit zumindest im Wesentlichen baugleich zur ersten Halteeinheit ausgebildet ist. So kann die zweite Halteeinheit insbesondere komplementär und/oder gespiegelt zur ersten Halteeinheit ausgebildet sein. Darüber hinaus kann die zweite Halteeinheit derart ausgebildet sein, dass die Schrägstellung und/oder die Verdrillung und/oder Tordierung der Strahlungsquellen zueinander sichergestellt werden kann.

Insbesondere ist vorgesehen, dass über die zweite Halteinheit keine Strom- und/oder Energiezuführung zu den Strahlungsquellen erfolgt. Die Energie- bzw. Stromversorgung der Strahlungsquellen kann insbesondere über in der ersten Halteeinheit vorgesehene Energieversorgungsleitungen gewährleitstet werden. Die zweite Halteeinheit dient letztlich zur Fixierung und/oder Sicherung bzw. zur stabilen Ausrichtung der Strahlungsquellen in dem Reflektor.

Die zweite Halteeinheit kann ferner an einem anderen Bereich, beispielsweise an der gegenüberliegenden Seite des Reflektors und/oder des Gehäuses, angeordnet sein.

Vorzugsweise ist die erste Halteeinheit und/oder das erste Verbindungsmittel mit einem ersten Verbindungsabschnitt verbunden. Der erste Verbindungsabschnitt kann mit dem Gehäuse und/oder mit dem Reflektor lösbar verbindbar sein. Insbesondere steht der erste Verbindungsabschnitt zumindest teilweise über die Außenseite des Gehäuses ab bzw. erstreckt sich entlang der Außenseite des Gehäuses. Der erste Verbindungsabschnitt ermöglicht somit die Verbindung der ersten Halteeinheit nach außen, insbesondre zu der Außenseite des Gehäuses, die der Innenseite des Reflektors abgewandt ist.

Ferner kann an dem ersten Verbindungsabschnitt außenseitig eine erste Versorgungseinrichtung, vorzugsweise aufweisend eine Mehrzahl von Vorschaltgeräten, insbesondere zum Betrieb der Strahlungsquellen, anordnenbar und/oder verbindbar sein. Somit kann der erste Verbindungsabschnitt auch als "Andockbereich" für Netzgeräte, Vorschaltgeräte und dergleichen dienen, die beispielsweise in einer Versorgungseinrichtung angeordnet sind. Die erste Versorgungseinrichtung kann ferner elektrisch über den ersten Verbindungsabschnitt mit der ersten Halteeinheit verbunden sein.

Darüber hinaus kann das Haltesystem bzw. die Halteeinrichtung auch modular durch verschiedene Verbindungsabschnitte ausgebildet sein, die insbesondere unmittelbar aneinander angeordnet und vorzugsweise miteinander fest und lösbar verbindbar sind. So kann beispielsweise die zweite Halteeinheit und/oder das zweite Verbindungsmittel mit einem zweiten Verbindungsabschnitt verbunden sein. Auch der zweite Verbindungsabschnitt kann mit dem Gehäuse und/oder dem Reflektor lösbar verbindbar sein. Vorzugsweise steht auch der zweite Verbindungsabschnitt gegenüber der Außenseite des Gehäuses ab. Der zweite Verbindungsabschnitt kann mit dem ersten Verbindungsabschnitt mittelbar oder unmittelbar verbunden sein.

Bei einer mittelbaren Verbindung kann vorgesehen sein, dass wenigstens ein weiterer Verbindungsabschnitt vorgesehen ist, der lösbar mit dem ersten und/oder zweiten Verbindungsabschnitt formschlüssig und/oder reibschlüssig und/oder kraftschlüssig verbindbar und/oder verbunden ist. Dies ermöglicht letztlich den modularen Aufbau der Halteeinrichtung und darüber hinaus auch eine vergleichsweise einfache Variation der Länge der Halteinrichtung, die insbesondere zur Länge der verwendeten Strahlungsquelle korrelieren kann.

Besonders bevorzugt ist, dass der erste und der zweite Verbindungsabschnitt derart ausgebildet sind, dass sie lösbar miteinander formschlüssig und/oder reibschlüssig und/oder kraftschlüssig verbindbar und/oder verbunden sind. Bei einer solchen Verbindung könnten der erste und der zweite Verbindungsabschnitt unmittelbar miteinander verbunden werden.

Zur Verbindung der Verbindungsabschnitte können Verriegelungsmittel vorgesehen sein.

Insbesondere ragt der erste, zweite und/oder weitere Verbindungsabschnitt zumindest teilweise in das Innere des Reflektors und/oder grenzt an die Innenseite des Reflektors an. Der erste, zweite und/oder weitere Verbindungsabschnitt kann auch gegenüber der Innenseite des Reflektors zurückversetzt sein, der Innenseite des Reflektors jedoch, vorzugsweise unmittelbar, zugewandt sein. Vorzugsweise weisen der erste, zweite und/oder weitere Verbindungsabschnitt an der dem Innenraum des Reflektors zugewandten Außenfläche eine reflektierende Oberfläche auf, die vorzugsweise eine gerichtete bzw. direkte Reflektion mit einem Reflexionsgrad von wenigstens 0,6, bevorzugt wenigstens 0,8, weiter bevorzugt wenigstens 0,9, aufweist.

Der Reflektor kann beispielsweise derart ausgebildet sein, dass er an einem Profil, insbesondere einem Alu-Profil, montierbar ist. So kann beispielsweise der Reflektor als Blech, vorzugsweise Aluminium-Blech, ausgebildet sein, wobei die Form des Reflektors durch Einspannen des Reflektors an dem entsprechenden Profil und/oder den Verbindungsabschnitten ermöglicht werden kann. Die Verbindungsabschnitte können bei einer solchen Ausbildung des Reflektors für die Stabilität der gesamten Bestrahlungsvorrichtung sorgen bzw. diese Stabilität sicherstellen.

Besonders bevorzugt sind zwischen 3 bis 25, vorzugsweise zwischen 4 bis 15, weiter bevorzugt zwischen 5 bis 10, Strahlungsquellen, erste Haltemittel und/oder zweite Haltemittel vorgesehen.

Die Anzahl der Haltemittel ist insbesondere in Abhängigkeit der Anzahl der Strahlungsquellen ausgebildet. In diesem Zusammenhang kann vorgesehen sein, dass ein "Überhang" an ersten oder zweiten Haltemitteln vorhanden ist, so dass beispielsweise nicht an jedem Haltemittel eine Strahlungsquelle angeordnet sein muss.

Ferner kann die Anzahl der Strahlungsquellen in Abhängigkeit der gewünschten UV-Strahlungsdosis, der Länge des Reflektors, des zu behandelnden Volumenstroms des Mediums und dergleichen ausgebildet werden. Durch den insgesamt ermöglichten modularen Aufbau der gesamten Bestrahlungsvorrichtung kann auch eine individuelle Anpassung der Bestrahlung erfolgen. Beispielsweise kann auch eine Hinzufügung von Strahlungsquellen bedarfsweise ermöglicht werden, indem beispielsweise an dem jeweiligen Verbindungsbereich der ersten und/oder zweiten Halteeinheit weitere Haltemittel angeordnet werden oder bereits vorhandene "überschüssige" Haltemittel zur Montage der Strahlungsquellen genutzt werden.

Vorzugsweise sind wenigstens zwei, bevorzugt wenigstens drei, insbesondere alle, Strahlungsquellen zueinander baugleich ausgebildet. Hierdurch wird ein verbessertes Strahlungsbild erreicht, da die Strahlungsquellen zumindest im Wesentlichen die gleiche Strahlung aussenden.

Insbesondere weist wenigstens eine, bevorzugt alle, Strahlungsquellen einen Durchmesser D zwischen 1 cm bis 20 cm, bevorzugt zwischen 2 cm bis 10 cm, weiter bevorzugt zwischen 4 cm bis 6 cm, auf. Der vorgenannte Durchmesser kann der mittlere und/oder der maximale Durchmesser der Strahlungsquelle sein. Insbesondere ist vorgesehen, dass der Durchmesser der Strahlungsquelle zumindest im Wesentlichen konstant ist.

Vorzugsweise weist wenigstens eine, bevorzugt alle, Strahlungsquellen eine Länge zwischen 0,2 m bis 10 m, bevorzugt zwischen 0,5 m bis 5 m, weiter bevorzugt zwischen 1 m bis 2 m, auf.

Darüber hinaus kann der innere Durchmesser des Reflektors, insbesondere der innere maximale Durchmesser des Reflektors, zwischen 100 bis 1000 cm, bevorzugt zwischen 200 bis 600 cm, betragen. Insbesondere ist bei einem inneren Durchmesser von 250 cm +/- 20 % vorgesehen, dass fünf Strahlungsquellen eingesetzt werden. Je größer der innere Durchmesser des Reflektors ausgebildet ist, desto mehr Strahlungsquellen können eingesetzt werden. So kann beispielsweise vorgesehen sein, dass bei einem inneren Durchmesser des Reflektors von 350 cm +/-20 % in etwa sieben Strahlungsquellen eingesetzt werden. Bei einem inneren Durchmesser des Reflektors von 500 cm +/- 20 % können beispielsweise zehn Strahlungsquellen eingesetzt werden. In diesem Zusammenhang ist vorgesehen, dass der innere Durchmesser des Reflektors insbesondere zumindest im Wesentlichen konstant ausgebildet ist - und zwar über die Längserstreckung des Reflektors.

Besonders bevorzugt ist, wenn eine Auswerteeinrichtung zur Erfassung wenigstens einer chemischen und/oder physikalischen Größe vorgesehen ist. Die Auswerteeinrichtung kann beispielsweise in dem ersten Verbindungsabschnitt und/oder in der ersten Halteeinheit und/oder in der zweiten Halteeinheit und/oder im ersten und/oder zweiten Verbindungsmittel angeordnet sein. Insbesondere kann die Auswerteeinrichtung wenigstens einen Temperatursensor, UV-Sensor und/oder Geschwindigkeitssensor aufweisen. Die Auswerteeinrichtung kann zur Verbesserung der Funktionsweise der Bestrahlung dienen. So können die durch die Auswerteeinrichtung erfassten chemischen und/oder physikalischen Größen zur Steuerung und/oder Regelung der Bestrahlung, des Volumentstroms des Mediums und dergleichen dienen, so dass insbesondere eine optimierte Betriebsweise bereitgestellt werden kann.

Die Länge des ersten Haltemittels und/oder des zweiten Haltemittels kann zwischen 0,5 • D_{R} bis 0,99 • D_{R}, bevorzugt zwischen 0,1 • D_{R} bis 0,5 • D_{R} betragen. In diesem Zusammenhang gibt D_{R} den inneren, insbesondere maximalen, Durchmesser des Reflektors an. Somit können die ersten und/oder zweiten Haltemittel sowohl mittig im Reflektor, als auch außerzentrisch im Reflektor angeordnet sein. Die Ausbildung der Länge der Haltemittel kann darüber hinaus auch in der jeweiligen Halteeinheit - das heißt in der ersten und/oder zweiten Halteeinheit - variieren.

Besonders bevorzugt ist wenigstens eine, weiter bevorzugt wenigstens zwei, insbesondere alle, Strahlungsquellen als LED ausgebildet. LEDs haben sich bei der Bestrahlung zur Abtötung von Mikroorganismen im Medium als besonders vorteilhaft herausgestellt, insbesondere da sie sich durch eine Langlebigkeit auszeichnen.

Bei einer weiteren ganz besonders bevorzugten Ausführungsform des Erfindungsgedankens ist vorgesehen, dass die Innenseite des Gehäuses und/oder des Reflektors ein Reflexionsgrad für die von der Strahlungsquelle emittierte UV-Strahlung von wenigstens 0,7, bevorzugt von wenigstens 0,8, weiter bevorzugt von wenigstens 0,9, aufweist. Insbesondere ist der Reflexionsgrad derart gewählt, dass die erfindungsgemäßen Interferenzen hervorgerufen werden können, die durch Wechselwirkung der an der Innenseite des Gehäuses reflektierten, vorzugsweise gerichteten, Strahlung mit der im Inneren des Gehäuses befindlichen Strahlung hervorgerufen werden können.

Besonders bevorzugt wird lediglich ein geringer Anteil der UV-Strahlung an der Innenwandung des Gehäuses bzw. des Reflektors transmittiert. Bevorzugt beträgt der transmittierte Anteil der UV-Strahlung weniger als 0,15, bevorzugt weniger als 0,1, weiter bevorzugt weniger als 0,05.

Die Innenwandung und/oder die Innenseite des Gehäuses und/oder des Reflektors kann mit einer UV-Strahlung reflektierenden Beschichtung, die insbesondere den gewünschten Reflexionsgrad der Innenseite sicherstellt, zumindest bereichsweise, vorzugsweise vollflächig bzw. vollständig, beschichtet sein. Alternativ oder zusätzlich kann vorgesehen sein, dass die Innenwandung und/oder die Innenseite des Gehäuses als Material Metall aufweist und/oder daraus besteht. Insbesondere kann die Innenwandung ein Blech, insbesondere ein Aluminium aufweisendes Blech und/oder ein verzinktes Blech und/oder ein Edelstahlblech, aufweisen und/oder daraus bestehen. Dabei kann die Innenwandung des Gehäuses als in das Gehäuse einsetzbare Komponente ausgebildet sein. Besonders bevorzugt ist ein mehrmals beschichtetes Aluminiumblech als Material für die Innenwandung des Gehäuses und/oder den Reflektor vorgesehen.

Vorzugsweise weist das Gehäuse und/oder der Reflektor eine Länge zwischen 30 bis 200 cm, bevorzugt zwischen 80 bis 150 cm, auf. Das Gehäuse kann einen Durchmesser, vorzugsweise einen Innendurchmesser, zwischen 8 bis 30 cm, bevorzugt zwischen 10 bis 20 cm, aufweisen. Bei einer besonders bevorzugten Ausführungsform weist das Gehäuse und/oder der Reflektor eine Länge zwischen 80 bis 150 cm und einen Innendurchmesser zwischen 10 bis 20 cm auf. Bei den vorgenannten Größenordnungen kann insbesondere für einen Mediumstrom mit einer Strömungsgeschwindigkeit zwischen 1 bis 2 m/s eine effiziente Inaktivierung der Mikroorganismen gewährleistet werden.

Darüber hinaus kann die erfindungsgemäße Bestrahlungseinrichtung auch in Klimaanlagen oder Bürogebäuden oder dergleichen eingesetzt werden. Dabei versteht es sich, dass die Bestrahlungsvorrichtung in Abhängigkeit des Mediumstromes und/oder des Durchsatzes des Mediumstromes ausgebildet sein kann.

Bei einer besonders bevorzugten Ausführungsform ist vorgesehen, dass die Strahlungsquelle eine langgestreckten Form mit einer Mehrzahl von Leuchtmitteln, vorzugsweise LEDs aufweist, die entlang eines "arrays" angeordnet sind.

Alternativ oder zusätzlich kann die Strahlungsquelle auch als UV-Niederdrucklampen, insbesondere eine Niederdruck-Quecksilber-Entladungslampe, und/oder als UV-Mitteldrucklampe ausgebildet sein.

Letztlich kann die Strahlungsquelle die zur Inaktivierung der Mikroorganismen benötigte UV-Strahlung, insbesondere die UV-C-Strahlung, bereitstellen. Dabei kann die Strahlungsquelle an ihrer Oberfläche eine Intensität zwischen 1000 bis 8000 W/m², bevorzugt zwischen 2000 bis 6000 W/m² und insbesondere von 4200 W/m² +/- 20 %, aufweisen.

Des Weiteren kann die Strahlungsquelle insbesondere eine Leistung von wenigstens 100 W, bevorzugt von 190 W +/- 10 %, erbringen. Die Leistung im Bereich der UV-C-Strahlung kann bevorzugt zwischen 10 bis 100 W, weiter bevorzugt zwischen 50 bis 70 W, betragen. Die von der Strahlungsquelle emittierte Strahlung kann mit ihrer Intensität im Abstandsquadrat abnehmen. Durch die erreichte konstruktive Interferenz kann dieser Reduzierung der Amplitude entgegengewirkt werden.

Vorzugsweise sind die mehreren Strahlungsquellen zumindest bereichsweise überlappend in dem Gehäuse angeordnet. Die Länge des Überlappungsbereiches von wenigstens zwei Strahlungsquellen kann wenigstens 20 %, bevorzugt wenigstens 30 %, weiter bevorzugt zwischen 30 % bis 100 %, der Länge wenigstens einer Strahlungsquelle entsprechen.

Bei einer weiteren bevorzugten Ausgestaltung ist vorgesehen, dass die Vorrichtung einen Ventilator zum Erzeugen des Mediumstromes vom Einlass zum Auslass umfasst. Der Ventilator ist insbesondere derart eingestellt, dass der Mediumstrom eine Durchströmungsgeschwindigkeit von 1 bis 5 m/s, bevorzugt zwischen 1 bis 2 m/s, aufweisen kann. Die Durchströmungsgeschwindigkeit kann insbesondere auch von dem Innendurchmesser des Gehäuses und/oder von der erreichten konstruktiven Interferenz in der UV-Behandlungskammer (das heißt im Innenraum des Gehäuses) abhängen.

Bevorzugt ist der Ventilator stromaufwärts zu der UV-Behandlungskammer bzw. zu der Strahlungsquelle und/oder dem Innenraum des Gehäuses angeordnet, um den Mediumstrom vom Einlass zu dem Auslass zu erzeugen.

Der Mediumstrom kann zumindest im Wesentlichen laminar durch den Innenraum des Gehäuses geführt werden. Alternativ oder zusätzlich kann aber auch vorgesehen sein, dass der in dem Gehäuse befindliche bzw. das Gehäuse durchströmende Mediumstrom eine turbulente Strömung ausbildet.

Des Weiteren kann mindestens eine Strahlungsquelle UV-Strahlung in einem Wellenlängenbereich von wenigstens 240 bis 300 nm, bevorzugt in einem Wellenlängenbereich von 250 bis 285 nm, weiter bevorzugt von 270 bis 280 nm und insbesondere von 254 nm +/- 10 % und/oder von 278 nm +/- 10 %, emittieren. Bei einer UV-C-Strahlung mit einer Wellenlänge von 254 nm +/- 10 % kann insbesondere eine hohe Vireninaktivierung erreicht werden. UV-Strahlung mit einer Wellenlänge in der vorgenannten Größenordnung ermöglicht erfindungsgemäß die Abtötung der Mikroorganismen in dem Mediumstrom.

Vorzugsweise ist vor dem Einlass ein Vorfilter angeordnet. Der Vorfilter kann bevorzugt derart ausgebildet sein, dass Partikel mit einem Durchmesser größer 1 µm, bevorzugt größer 0,5 µm, aus dem Mediumstrom herausgefiltert werden. Somit können insbesondere aus dem Mediumstrom solche Partikel herausgefiltert werden, die andernfalls eine sogenannte "Schattenbildung" in der UV-Behandlungskammer bei der entstehenden Wechselwirkung mit der UV-Strahlung erzeugen würden. Letztlich ist in diesem Zusammenhang relevant, dass die UV-Strahlung in einer Größenordnung zwischen 0,2 bis 0,3 µm liegt. Da die vorgenannten Partikel, beispielsweise Staubpartikel oder Pollen oder dergleichen, jedoch einen größeren Durchmesser als die Wellenlänge aufweisen, kann die Wellenlänge insbesondere nicht die Partikel mit einem Durchmesser von größer als 1 µm passieren. Ein Bakterium kann beispielsweise einen Durchmesser von circa 0,3 µm aufweisen. Erfindungsgemäß ist festgestellt worden, dass der Schatteneffekt vor Partikeln mit einem Durchmesser unter 0,5 µm, insbesondere zwischen 0,3 µm bis 0,5 µm, zwar vorhanden ist, jedoch noch die erreichte Abtötung der Mikroorganismen toleriert werden kann. So können auch diejenigen Mikroorganismen mit einem Durchmesser größer als die Wellenlänge der UV-Strahlung ebenfalls unschädlich gemacht werden, da auch auf sie die UV-Strahlung trifft.

Im Übrigen versteht es sich, dass in den vorgenannten Intervallen und Bereichsgrenzen jegliche Zwischenintervalle und Einzelwerte enthalten und als erfindungswesentlich offenbart anzusehen sind, auch wenn diese Zwischenintervalle und Einzelwerte nicht konkret angegeben sind.

Weitere Merkmale, Vorteile und Anwendungsmöglichkeiten der vorliegenden Erfindung ergeben sich aus der nachfolgenden Beschreibung von Ausführungsbeispielen anhand der Zeichnung und der Zeichnung selbst. Dabei bilden alle beschriebenen und/oder bildlich dargestellten Merkmale für sich oder in beliebiger Kombination den Gegenstand der vorliegenden Erfindung, unabhängig von ihrer Zusammenfassung in den Ansprüchen oder deren Rückbeziehung.

Es zeigt:
- Fig. 1: eine schematische perspektivische Darstellung eines erfindungsgemäßen Gehäuses,
- Fig. 2: eine schematische Darstellung einer erfindungsgemäßen Bestrahlungsvorrichtung,
- Fig. 3: eine schematische perspektivische Darstellung einer weiteren Ausführungsform einer erfindungsgemäßen Bestrahlungsvorrichtung,
- Fig. 4: eine schematische perspektivische Darstellung einer weiteren Ausführungsform einer erfindungsgemäßen Bestrahlungsvorrichtung,
- Fig. 5: eine schematische Seitenansicht auf die in Fig. 9 dargestellte Bestrahlungsvorrichtung,
- Fig. 6: eine weitere schematische Seitenansicht auf die in Fig. 9 dargestellte Bestrahlungsvorrichtung,
- Fig. 7: eine schematische Draufsicht auf eine erfindungsgemäße erste Halteeinheit,
- Fig. 8: eine schematische Darstellung einer erfindungsgemäßen Halteeinrichtung,
- Fig. 9: eine schematische Darstellung der Anordnung wenigstens einer Strahlungsquelle im Reflektor,
- Fig. 10: eine schematische Darstellung der Anordnung von zwei Strahlungsquellen im Reflektor,
- Fig. 11: eine schematische Darstellung einer weiteren Ausführungsform eines ersten Haltemittels und
- Fig. 12: eine schematische Darstellung der erfindungsgemäßen Ausrichtung einer Strahlungsquelle.

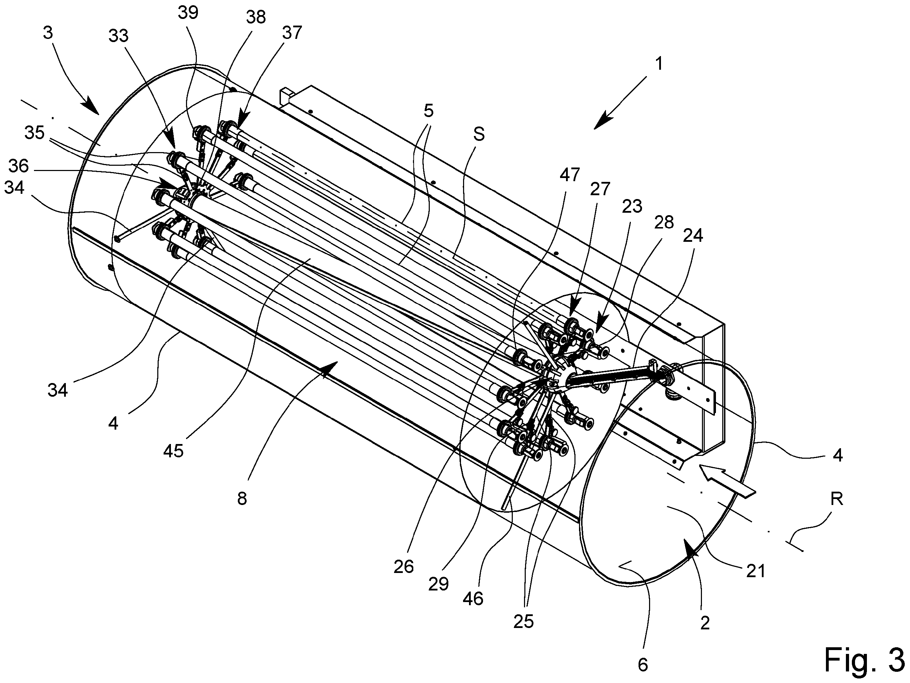

Fig. 3 zeigt eine Bestrahlungsvorrichtung 1 zur UV-Strahlung, insbesondere zur UV-C-Bestrahlung, eines die Bestrahlungsvorrichtung 1 durchströmenden Mediums. Als Medium kann Wasser oder Luft vorgesehen sein. In weiteren Ausführungsformen kann als Medium auch ein Gasgemisch und/oder ein Dampfgemisch verwendet werden.

Die Bestrahlungsvorrichtung 1 wird insbesondere zur Inaktivierung bzw. Abtötung von in dem Medium befindlichen Mikroorganismen, wie Bakterien, Keime, Schimmel und/oder Viren, eingesetzt.

Die Bestrahlungsvorrichtung 1 weist einen Einlass 2 und einen Auslass 3 auf. Der Einlass 2 dient zum Einlass des Mediums und der Auslass 3 zum Auslass des Mediums aus der Bestrahlungsvorrichtung 1. Die Bestrahlungsvorrichtung 1 umfasst ferner ein Gehäuse 4, das den Einlass 2 und den Auslass 3 aufweist und das von dem Medium durchströmt wird. In dem Gehäuse 4 ist wenigstens eine Strahlungsquelle 5 angeordnet. Die Strahlungsquelle 5 kann mit dem Gehäuse 4 verbunden und/oder befestigt und/oder an dem Gehäuse 4 gelagert sein. Fig. 3 zeigt, dass die zwei Stirnseiten der Strahlungsquelle 5 an dem Gehäuse 4 befestigt sind.

Das Gehäuse 4 weist einen Reflektor 21 auf, wobei der Reflektor 21 an der der Strahlungsquelle 5 zugewandten Innenseite 6 zumindest bereichsweise, vorzugsweise vollflächig, reflektierend mit einem Reflexionsgrad für die von der Strahlungsquelle 5 emittierte UV-Strahlung, insbesondere der UV-C-Strahlung, von wenigstens 0,6 ausgebildet ist. In dem in Fig. 3 dargestellten Ausführungsbeispiel ist vorgesehen, dass der Reflexionsgrad wenigstens 0,7, bevorzugt wenigstens 0,8, beträgt.

Die Strahlungsquelle 5 ist derart in dem Gehäuse 4 angeordnet, dass die von der Strahlungsquelle 5 emittierte Strahlung an der Innenseite 6 des Reflektors 21, vorzugsweise gerichtet, reflektiert wird.

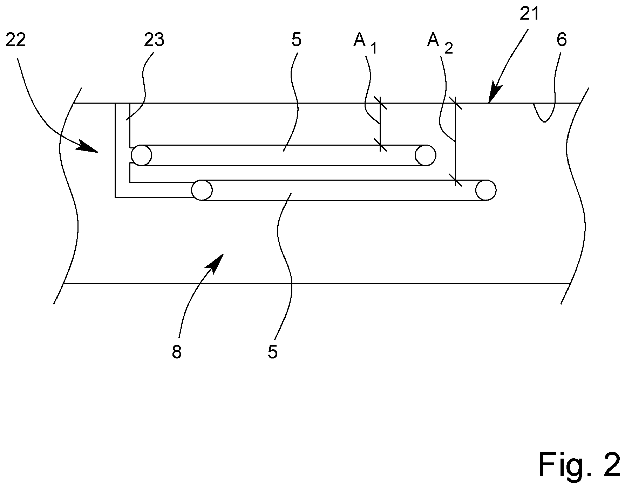

Fig. 2 zeigt, dass eine Halteeinrichtung 22 vorgesehen ist, die eine erste Halteeinheit 23 umfasst. Alle Strahlungsquellen 5 sind an der Halteeinrichtung 22, insbesondere an der ersten Halteeinheit 23, fixiert bzw. gehalten. Die erste Halteeinheit 23 ist im dargestellten Ausführungsbeispiel einstückig ausgebildet. In weiteren Ausführungsformen kann die erste Halteeinheit 23 auch mehrteilig ausgebildet sein.

Weiter zeigt Fig. 2, dass die wenigstens zwei Strahlungsquellen 5 derart an der Halteeinrichtung 22 angeordnet sind, dass die jeweiligen minimalen (lichten) Abstände A₁ und A₂ von der jeweiligen Strahlungsquelle zur Innenseite 6 des Reflektors 21 sich voneinander unterscheiden. Trotzdem sind beide Strahlungsquellen 5 an einer gemeinsamen ersten Halteeinheit 23 angeordnet und können insbesondere gemeinsam gehandhabt werden.

Die minimalen Abstände A₁ und A₂ von der jeweiligen Strahlungsquelle zur Innenseite 6 des Reflektors 21 können sich insbesondere um wenigstens 15 % voneinander unterscheiden.

Fig. 3 zeigt eine weitere erfindungsgemäße Ausgestaltung einer Bestrahlungsvorrichtung 1. Bei der in Fig. 3 gezeigten Ausführungsform ist vorgesehen, dass die Mittelachsen S zumindest im Wesentlichen parallel zur Mittelachse R des Reflektors 21 angeordnet sind. Die erste Halteeinheit 23 ist über ein erstes Verbindungsmittel 24 mit dem Gehäuse 4 bzw. dem Reflektor 21 verbunden, wobei die erste Halteeinheit 23 wenigstens drei erste Haltemittel 25 umfasst. Die ersten Haltemittel 25 sind als langgestreckte, stegförmige Haltearme, insbesondere als Tragarme, ausgebildet. Ferner sind die ersten Haltemittel 25 zueinander beabstandet.

Fig. 3 zeigt weiter, dass die ersten Haltemittel 25 mit einem Endbereich 28 an einem ersten, mit dem ersten Verbindungsmittel 24 verbundenen Verbindungsbereich 26 angeordnet, insbesondere gelagert und/oder verbunden, sind. Die Strahlungsquellen 5 sind mit ihrem stirnseitigen Endbereich 27 an dem freien - d.h. nicht gelagerten - Endbereich 29 der ersten Haltemittel 25 befestigt. Zur Befestigung können Befestigungsmittel 47 vorgesehen sein. Insbesondere sind die Strahlungsquellen lösbar sowie reibschlüssig und/oder formschlüssig an dem ersten Haltemittel 25 befestigt.

In weiteren Ausführungsformen kann vorgesehen sein, dass wenigstens zwei Strahlungsquellen 5 derart in dem und/oder an der ersten Halteeinheit angeordnet und/oder gelagert sind, dass sie entlang der jeweiligen Längsrichtung der wenigstens zwei Strahlungsquellen 5 verlaufenden Mittelachsen S miteinander einen Winkel einschließen, bevorzugt zwischen 10° bis 90°.

Die Innenseite 6 des Reflektors 21 kann auch Bestandteil einer Innenwandung sein. Insbesondere ist der Reflektor 21 als Innenwandung ausgebildet. Die Innenwandung kann in weiteren, nicht dargestellten Ausführungsbeispielen als austauschbare Komponente bzw. als aus dem Gehäuse 4 entnehmbare Komponente ausgebildet sein.

Im Innenraum des Gehäuses 4 bzw. in der im Inneren gebildeten Behandlungskammer 8 kann somit ein Interferenzbild bzw. ein Interferenzmuster erzeugt werden, das insbesondere durch konstruktive Interferenz charakterisiert wird.

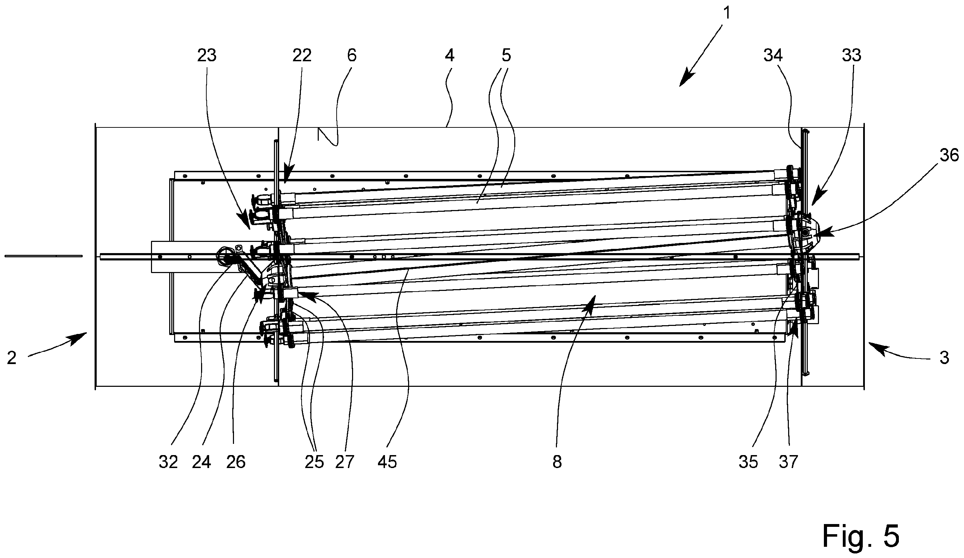

Die Innenseite 6 weist bei dem in Fig. 5 dargestellten Ausführungsbeispiel einen Reflexionsgrad von wenigstens 0,7 auf. In weiteren Ausführungsformen kann der Reflexionsgrad wenigstens 0,8, insbesondere wenigstens 0,9, betragen. Der Reflexionsgrad gibt letztlich denjenigen Anteil der von der Strahlungsquelle 5 emittierten Strahlung an, der an der Innenseite 6 reflektiert wird.

Wenngleich es nicht dargestellt ist, kann bei weiteren Ausführungsformen vorgesehen sein, dass die Innenseite 6 des Gehäuses 4 und/oder die Innenwandung bzw. der Reflektor 21 mit einer UV-Strahlung reflektierenden Beschichtung zumindest bereichsweise, insbesondere vollständig, beschichtet ist.

Des Weiteren ist nicht dargestellt, dass der Reflektor 21 als Material Metall aufweisen kann und/oder daraus bestehen kann. In weiteren Ausführungsformen kann die Innenwandung bzw. der Reflektor 21 ein Blech, insbesondere ein Aluminiumblech und/oder ein verzinktes Blech und/oder ein Edelstahlblech, sein.

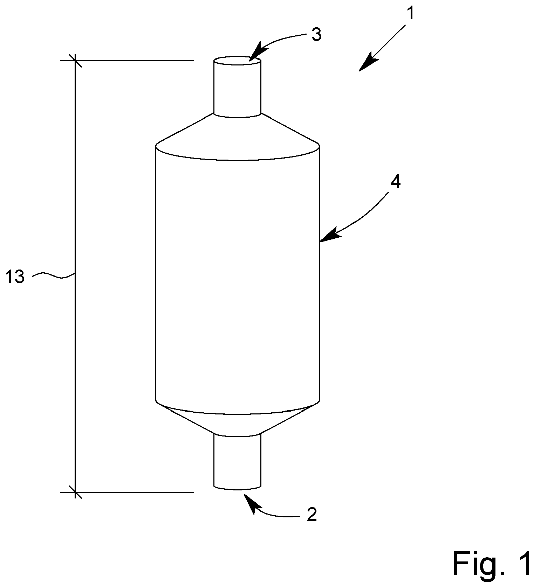

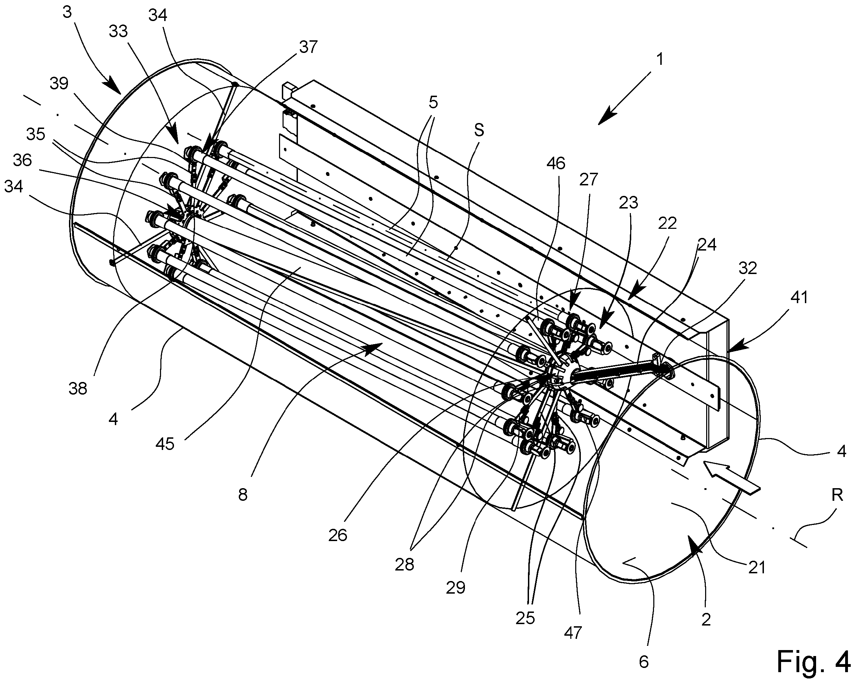

In den Fig. 4 bis 12 ist eine weitere Ausführungsform einer Bestrahlungsvorrichtung 1 dargestellt. In diesem Zusammenhang versteht es sich, dass Ausführungen, wie sie zur Bestrahlungsvorrichtung 1 nach den Fig. 1 bis 3 gemacht worden sind, auch für die nachfolgend beschriebene weitere Ausführungsform der Bestrahlungsvorrichtung 1 gelten können - jedoch nicht gelten müssen. Zur Vermeidung von unnötigen Wiederholungen wird darauf verzichtet, auf die diesbezüglichen einzelnen Merkmale erneut einzugehen.

Die in den Fig. 4 bis 6 dargestellte Ausführungsform unterscheidet sich insbesondere von der in Fig. 3 dargestellten Ausführungsform darin, dass die Mittelachsen S der Strahlungsquellen 5 einen Winkel α zur Mittelachse R des Reflektors 21 einschließen. Die weiteren Merkmale - die in Bezug zur jeweiligen Ausführungsform beschrieben worden sind - können bei der jeweiligen anderen Ausführungsform nach Fig. 3 bzw. nach Fig. 4 realisiert werden.

Die Fig. 4 zeigt eine Bestrahlungsvorrichtung 1, die zur UV-Bestrahlung, insbesondere UV-C-Bestrahlung, eines die Bestrahlungsvorrichtung 1 durchströmenden Mediums ausgebildet ist. Das Medium kann ein Fluid oder ein Gas sein. Insbesondere ist als Medium Wasser oder Luft vorgesehen. Die Bestrahlungsvorrichtung 1 wird zur Inaktivierung von in dem Medium befindlichen Mikroorganismen, wie Bakterien, Keime, Schimmel und/oder Viren, verwendet. Insbesondere wird die Bestrahlungsvorrichtung 1 zur Inaktivierung von Corona-Viren verwendet. Als Corona-Viren werden insbesondere SARS-CoV-2-Viren verstanden.

Die Bestrahlungsvorrichtung 1 weist ein Gehäuse 4 auf, das für das Medium einen Einlass 2 und einen Auslass 3 aufweist. In Fig. 4 ist die Durchströmungsrichtung des Mediums schematisch über Strömungs-Pfeile dargestellt.

Wenigstens eine Strahlungsquelle 5 ist in dem Gehäuse 4, nämlich im Inneren des Gehäuses 4, angeordnet. Das Innere des Gehäuses 4 umfasst die Behandlungskammer 8, in der die Strahlungsquelle(n) 5 angeordnet ist/sind.

Die Strahlungsquelle 5 dient zur Bestrahlung des das Gehäuse 4 durchströmenden Mediums.

In der Ausführungsform nach Fig. 4 ist eine Mehrzahl von Strahlungsquellen 5 vorgesehen.

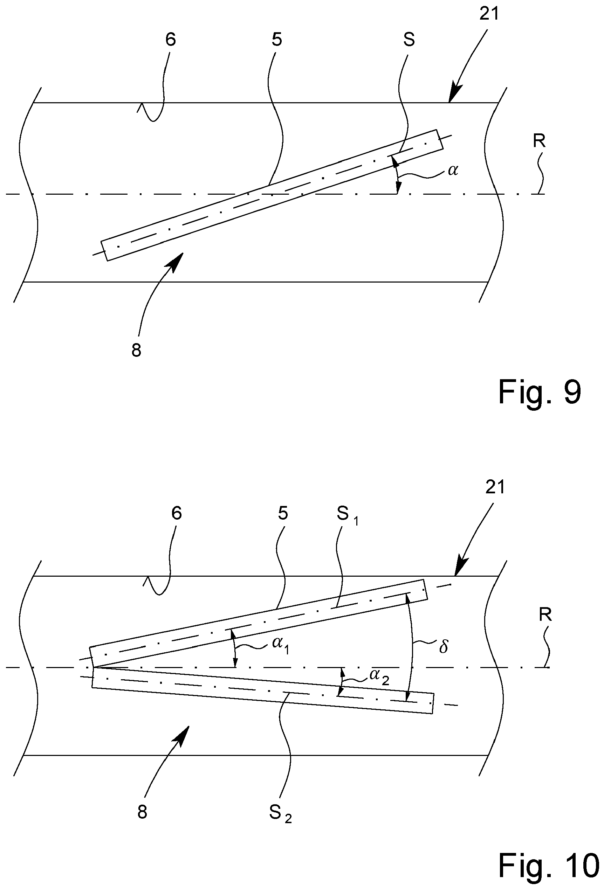

Das Gehäuse 4 weist einen Reflektor 21 auf. Die Innenseite 6 des Reflektors 21 bildet auch die Innenseite 6 des Gehäuses 4. In den dargestellten Ausführungsformen gemäß den Fig. 9 bis 11 ist aus Veranschaulichungsgründen der Reflektor 21 "durchsichtig" dargestellt.

Insbesondere ist der Reflektor 21 als ein Aluminium-Blech ausgebildet, das in einem entsprechenden Profil eingefasst bzw. gehalten werden kann.

Die Innenseite 6 ist zumindest bereichsweise, vorzugsweise vollflächig, reflektierend mit einem Reflexionsgrad für die von der Strahlungsquelle 5 emittierte UV-Strahlung von größer als 0,6 ausgebildet. Insbesondere ist die Innenseite 6 so ausgebildet, dass eine gerichtete, direkte Reflektion der Strahlung erfolgen kann. Hierzu ist die Innenseite 6 insbesondere glatt und eben ausgebildet.

Die Strahlungsquelle 5 wird mit einer Halteeinrichtung 22 gehalten und/oder fixiert. Die Halteeinrichtung 22 ist, vorzugsweise lösbar, mit dem Gehäuse 4 und/oder dem Reflektor 21 verbunden.

Fig. 4 zeigt, dass die Halteeinrichtung 22 derart ausgebildet ist, dass die Mittelachse S der wenigstens einen Strahlungsquelle 5 einen Winkel α zur Mittelachse R des Reflektors 21 einschließt.

Fig. 9 zeigt schematisch, dass die Strahlungsquelle 5 so angeordnet ist, dass zwischen den Mittelachsen S und R ein Winkel α eingeschlossen wird. Aus Veranschaulichungsgründen ist in Fig. 9 die Halteeinrichtung 22 nicht näher dargestellt.

Bei der in Fig. 9 dargestellten Ausführungsform liegt der eingeschlossene Winkel α zwischen der Mittelachse S der wenigstens einen Strahlungsquelle 5 und der Mittelachse R der Reflektors 21 zwischen arcsin((0,2 • D/L) und arcsin((4 • D)/L). Insbesondere liegt der Winkel α zwischen 2° ± 0,5°. Um die Schrägstellung der Strahlungsquelle 5 aus schematischen Gründen besser darzustellen, ist in den dargestellten Ausführungsformen nach Fig. 9 und 10 der Winkel α bewusst größer gewählt worden. Es versteht sich jedoch, dass diese Figuren als schematische Darstellungen zu verstehen sind und nicht die tatsächlichen Größenverhältnisse widerspiegeln.

Ferner versteht es sich, dass der Winkel α insbesondere in der vorgenannten Größenordnung liegt.

Vorzugsweise liegt der Winkel α zwischen arcsin(D/L) und arcsin((2 • D)/L). Somit beträgt der insgesamt eingenommen Schrägversatz insbesondere zwischen D und 2D.

Dabei gibt D den, insbesondere maximalen und/oder mittleren, Durchmesser der Strahlungsquelle 5 und L die Länge der Strahlungsquelle 5 an.

Die in den dargestellten Ausführungsformen gezeigten Strahlungsquellen 5 sind insbesondere als LED-Strahler ausgebildet.

Die Strahlungsquellen 5 sind ferner stabförmig bzw. zylinderförmig sowie langgestreckt ausgebildet. Die Längserstreckung der Strahlungsquelle 5 verläuft dabei zumindest im Wesentlichen in Richtung Längserstreckung des Reflektors 21 - unter Berücksichtigung der zuvor diskutierten Schrägstellung der Strahlungsquelle(n) 5. Somit wird bevorzugt keine orthogonale Anordnung der Strahlungsquelle 5 in Bezug zur Mittelachse R des Reflektors 21 vorgesehen.

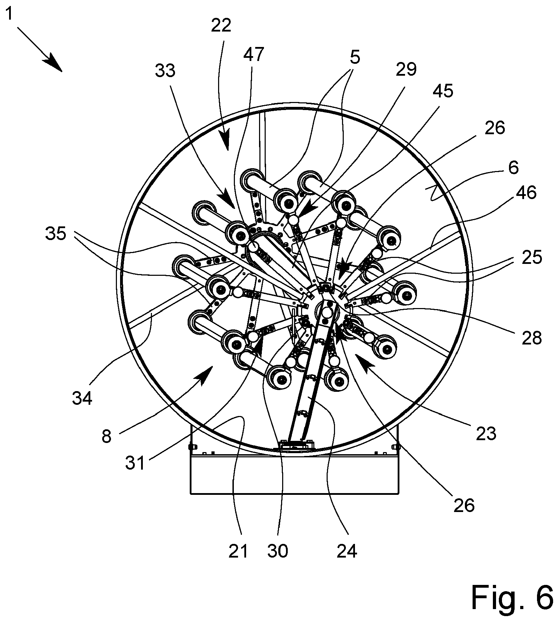

Wie zuvor erläutert, zeigen die Fig. 4 bis 6, dass eine Mehrzahl von Strahlungsquellen 5 an der Halteeinrichtung 22 gehalten und/oder fixiert sind. Die Fig. 5 und 6 zeigen entsprechende Seitenansichten auf die in Fig. 4 dargestellte Bestrahlungsvorrichtung 1. So zeigt Fig. 6 den Einlass 2, wobei Fig. 5 die schräge Anordnung der Strahlungsquellen 5 durch die entsprechende Seitenansicht der Längsseite verdeutlicht.

In diesem Zusammenhang versteht es sich, dass in weiteren Ausführungsformen auch eine Mehrzahl von Halteeinrichtungen 22 vorgesehen sein kann, wobei an den jeweiligen Halteeinrichtungen 22 jeweils wenigstens eine Strahlungsquelle 5, bevorzugt eine Mehrzahl von Strahlungsquellen 5, befestigt sein kann. Diese Halteeinrichtungen 22 können dabei untereinander und/oder nebeneinander, insbesondere beabstandet zueinander, angeordnet sein. Besonders bevorzugt ist jedoch, dass eine einzige Halteeinrichtung 22 vorgesehen ist.

Die an der Halteeinrichtung 22 befestigten Strahlungsquellen 5 können insgesamt auch als "Lampenpaket" bzw. Strahlungseinheit bezeichnet werden.

Der Einlass 2 und der Auslass 3 können auch an anderen Stellen des Gehäuses 4 angeordnet sein. Letztlich dient der Einlass 2 zur Einführung des Mediums in die Behandlungskammer 8, während der Auslass 3 den Austritt des Mediums aus der Bestrahlungsvorrichtung 1 ermöglicht. Grundsätzlich kann erfindungsgemäß auch vorgesehen sein, dass eine Mehrzahl von Einlässen 2 und/oder eine Mehrzahl von Auslässen 3 vorhanden ist.

In der dargestellten Ausführungsform ist es so, dass in Längsrichtung des Reflektors 21 an der Halteeinrichtung 22 jeweils nur eine Strahlungsquelle 5 angeordnet ist. Die weiteren Strahlungsquellen 5 sind ebenfalls zumindest im Wesentlichen in Längsrichtung ausgerichtet. Nicht dargestellt ist, dass bei einer weiteren Ausführungsform auch vorgesehen sein kann, dass wenigstens zwei Strahlungsquellen 5 in Längsrichtung des Reflektors 21 hintereinander an einer Halteeinrichtung 22 angeordnet sein können. Auch kann eine Strahlungsquelle 5 mehrteilig ausgebildet sein.

Bei der in Fig. 5 dargestellten Ausführungsform ist es so, dass jede Mittelachse S jeder Strahlungsquelle 5 einen Winkel α zu der Mittelachse R des Reflektors 21 einschließt. In Fig. 10 ist schematisch dargestellt, dass die Mittelachsen S₁ und S₂ jeweils einen Winkel α₁ und α₂ zur Mittelachse R des Reflektors 21 einschließen.

Unter der Mittelachse wird diejenige Achse verstanden, die eine annähernde Symmetrieachse des Körpers bildet. Allerdings werden auch nicht-symmetrische Körper berücksichtigt. In diesem Fall kann die Mittelachse insbesondere durch den Schwerpunkt des Körpers und in Längserstreckung des Körpers verlaufen. Auch Abweichungen zur Mittelachse von ± 10% werden erfindungsgemäß noch unter der "Mittelachse" subsummiert.

In Fig. 6 ist schematisch dargestellt, dass die Mittelachsen S der Strahlungsquellen 5 zueinander zumindest im Wesentlichen parallel angeordnet sind.

In Fig. 10 ist schematisch dargestellt, dass wenigstens zwei Mittelachsen S₁ und S₂ zueinander versetzt, insbesondere schräg, angeordnet sind. Dabei kann der eingeschlossene Winkel δ zwischen wenigstens zwei Strahlungsquellen 5 zwischen 1° bis 50°, insbesondere zwischen 10° bis 40°, betragen.

Insbesondere können die Mittelachsen S der Strahlungsquellen 5 zueinander auch schräg und/oder windschief angeordnet sein.

Bei der in Fig. 4 dargestellten Halteeinrichtung 22 ist vorgesehen, dass diese derart ausgebildet ist, dass die Strahlungsquelle 5 bzw. die Strahlungsquellen 5 lösbar mit der Halteeinrichtung 22 verbunden sind.

Die Strahlungsquellen 5 können zueinander gleich beabstandet sein. Es kann jedoch auch vorgesehen sein, dass die Mittelachsen R einen unterschiedlichen Winkel α₁, α₂ zu der Mittelachse R des Reflektors 21 einschließen, wie dies schematisch beispielsweise in Fig. 10 dargestellt ist. Auch bei der in Fig. 10 dargestellten Ausführungsform ist vorgesehen, dass die Winkel α₁ und α₂ - aus schematischen Darstellungszwecken - bewusst "größer" dargestellt sind, um letztlich das Prinzip zu verdeutlichen.

Fig. 4 zeigt, dass die Halteeinrichtung 22 eine erste Halteeinheit 23 aufweist. Die erste Halteeinheit 23 ist über ein erstes Verbindungsmittel 24 der Halteeinrichtung 22 lösbar mit dem Gehäuse 4 und dem Reflektor 21 verbunden. Zur weiteren Stabilität der ersten Halteeinheit 23 sind darüber hinaus noch Haltestreben 46 vorgesehen, die jeweils mit dem Gehäuse 4 und/oder dem Reflektor 21 verbunden sind. Die Haltestreben 46 können als Bestandteil des ersten Verbindungsmittels 24 angesehen werden.

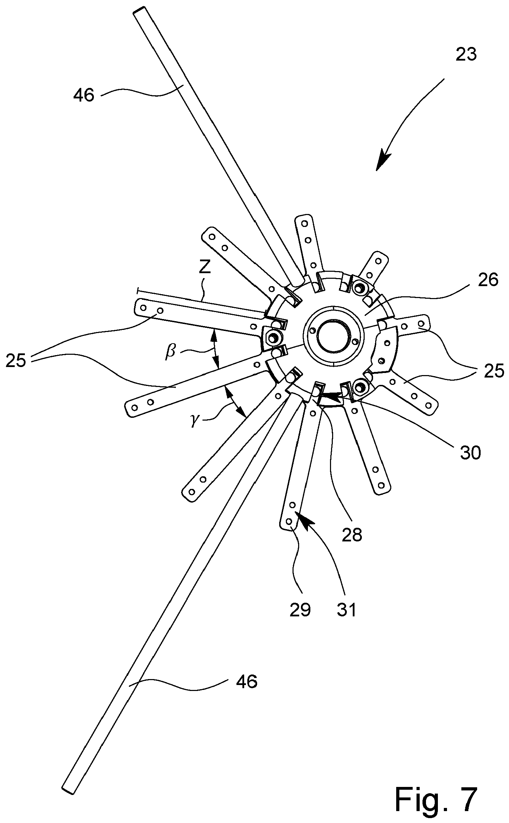

Schematisch sind die Haltestreben 46 darüber hinaus auch in Fig. 7 dargestellt. Fig. 7 zeigt die erste Halteeinheit 23 ohne entsprechende Befestigungsmittel 47 für die Strahlungsquellen 5.

Fig. 7 zeigt, dass die erste Halteeinheit 23 erste Haltemittel 25 aufweist, wobei die ersten Haltemittel 25 insbesondere als stegförmige Haltearme ausgebildet sind. Die ersten Haltemittel 25 können zumindest bereichsweise zueinander beabstandet sein, wie dies aus Fig. 7 ersichtlich ist. Die Beabstandung zwischen den ersten Haltemitteln 25 kann ferner variieren. Ebenfalls kann der eingeschlossene Winkel β, γ zwischen zwei unmittelbar benachbarten ersten Haltemitteln 25 variieren. Die Winkel β, γ beziehen sich insbesondere auf die Mittelachse der ersten Haltemittel 25.

Zur Befestigung der Strahlungsquellen 5 kann das erste Haltemittel 25 Befestigungsmittel 47 aufweisen. Die Befestigungsmittel 47 sind schematisch in Fig. 4 dargestellt.

Als Befestigungsmittel 47 kann beispielsweise ein Clip, ein Federschenkel und/oder eine Spannklemme vorgesehen sein. Letztlich sind unterschiedliche Befestigungsmittel 47 möglich. Das Befestigungsmittel 47 ist insbesondere ein Bestandteil des ersten Haltemittels 25.

In Fig. 6 ist dargestellt, dass die ersten Haltemittel 25 mit einem ersten Verbindungsbereich 26 der ersten Halteeinheit 23 verbunden sind. Ausgehend von diesem Verbindungsbereich 26 stehen die ersten Haltemittel 25 ab. Die ersten Haltemittel 25 sind mit dem einen Endbereich 28 mit dem Verbindungsbereich 26 verbunden. Die ersten Haltemittel 25 umfassen ferner einen weiteren Endbereich 29, der wiederum zur Anordnung der Strahlungsquellen 5, insbesondere der stirnseitigen Endbereiche 27 der Strahlungsquelle 5, vorgesehen ist. Somit können die ersten Haltemittel 25 insbesondere als Tragarm bzw. Kragarm ausgebildet sein. Der freie Endbereich 29 kann insbesondere nicht gelagert bzw. frei angeordnet sein. Der Endbereich 28 der ersten Haltemittel 25 kann dabei an dem ersten Verbindungbereich 26 unmittelbar angeordnet sein.

Insbesondere ergibt sich eine zumindest im Wesentlichen sternförmige bzw. sonnenförmige Ausbildung der ersten Halteeinheit 23, wie dies in Fig. 7 schematisch dargestellt ist.

Der Endbereich 28 kann an dem Verbindungsbereich 26 gelagert oder mit diesem fest verbunden sein. Auch kann vorgesehen sein, dass der Verbindungsbereich 26 und der Endbereich 28 einteilig miteinander ausgebildet sind.

In der dargestellten Ausführungsform ist ferner vorgesehen, dass ein erstes Verstellmittel 30 an dem Endbereich 28 angeordnet ist. Dieses erste Verstellmittel 30 ermöglicht eine relative Verstellung zum Verbindungsbereich 26 und insbesondere eine Verstellung der an dem jeweiligen ersten Haltemittel 25 befestigten Strahlungsquelle 5 - und zwar eine Verstellung der Mittelachse S der Strahlungsquelle 5 in Bezug zur Mittelachse R des Reflektors 21.

Nicht näher dargestellt ist, dass die ersten Haltemittel 25 zumindest bereichsweise auch teleskopierbar ausgebildet sind.

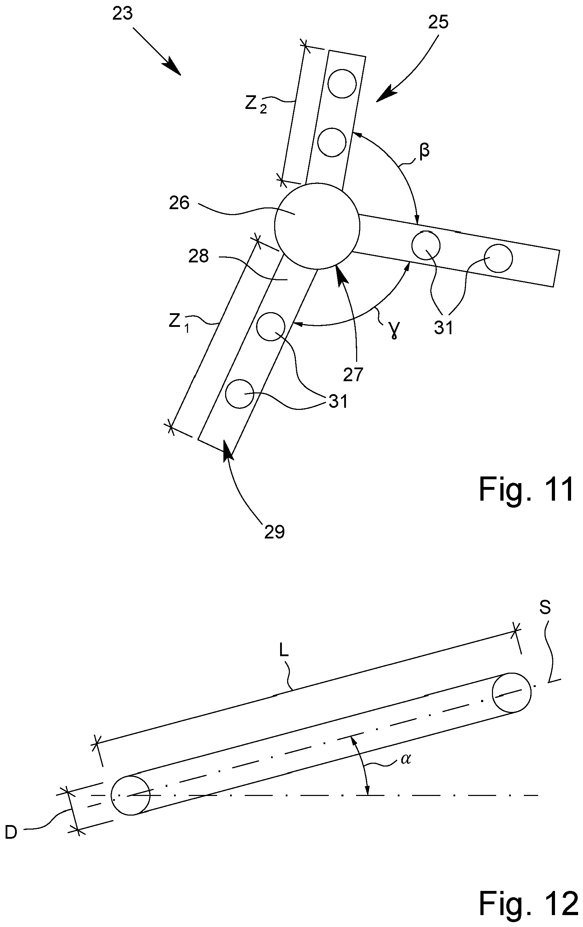

In Fig. 7 ist dargestellt, dass die ersten Haltemittel 25 eine unterschiedliche Länge Z aufweisen. Dies ist schematisch auch in Fig. 11 dargestellt.

In Fig. 6 ist schematisch dargestellt, dass die Strahlungsquelle 5 an dem einen stirnseitigen Endbereich 27 lösbar und reibschlüssig mit dem ersten Haltemittel 25, insbesondere mit dem Befestigungsmittel 47, verbunden ist.

Fig. 11 zeigt, dass die ersten Haltemittel 25 langgestreckt ausgebildet sind und dass wenigstens zwei erste Haltemittel 25 eine sich voneinander unterscheidende Länge Z₁, Z₂ aufweisen. Schematisch ist in Fig. 11 weiter dargestellt, dass je Haltemittel 25 eine Mehrzahl von Anordnungsbereichen 31 vorgesehen ist. Die Anordnungsbereiche 31 können zur Anordnung von Befestigungsmitteln 47 oder zur (unmittelbaren) Anordnung des stirnseitigen Endbereichs 27 der Strahlungsquelle 5 ausgebildet sein. So kann beispielsweise die Stirnseite der Strahlungsquelle 5 über den Anordnungsbereich 31 und somit auch über das Haltemittel 25 überstehen, insbesondere wenn der stirnseitige Endbereich 27 zumindest bereichsweise in dem Anordnungsbereich 31 aufgenommen und in diesem, vorzugsweise reibschlüssig, gehalten ist. Letztlich sind unterschiedliche Befestigungsmöglichkeiten zwischen der Strahlungsquelle 5 und dem ersten Haltemittel 25 möglich.

Die zwischen zwei unmittelbar benachbarten ersten Haltemittel 25 eingeschlossenen Winkel β, γ können insbesondere zueinander um wenigstens 5% abweichen, wie dies schematisch in Fig. 11 dargestellt ist.

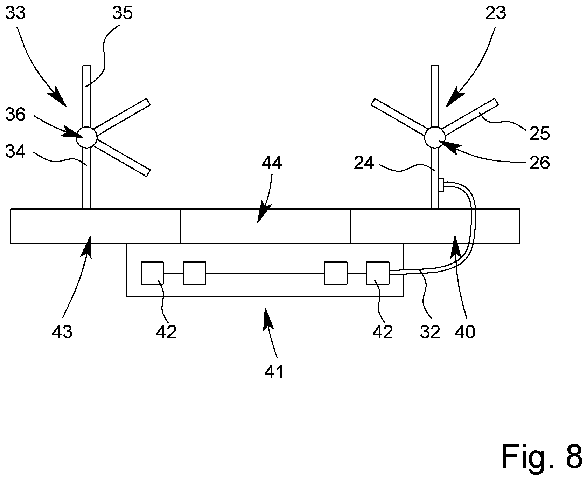

In Fig. 4 ist schematisch dargestellt, dass Energieversorgungsleitungen 32 zur Energieversorgung der Strahlungsquellen 5 vorgesehen sind. Diese Energieversorgungsleitungen 32 werden insbesondere entlang des ersten Verbindungsmittels 24 und insbesondere entlang der ersten Haltemittel 25 geführt. Die Energieversorgungsleitungen 32 können mit entsprechenden Netzteilen und/oder Vorschaltgeräten 42 verbunden sein, wie dies schematisch aus Fig. 8 ersichtlich wird. Insbesondere ist eine erste Versorgungseinrichtung 41 außerhalb des Gehäuses 4 an der Außenseite des Gehäuses 4, die der Innenseite 6 abgewandt ist, angeordnet.

Letztlich kann die erste Halteeinheit 23 zur Energiezuführung zu den Strahlungsquellen 5 ausgebildet sein.

In Fig. 4 ist dargestellt, dass eine zweite Halteeinheit 33 vorgesehen ist. Die zweite Halteeinheit 33 ist über ein zweites Verbindungsmittel 24 der Halteeinrichtung 22 lösbar mit dem Gehäuse 4 und dem Reflektor 21 verbunden.

Das zweite Verbindungsmittel 24 umfasst nach der in Fig. 4 dargestellten Ausführungsform wenigstens zwei Haltestreben, die die zweite Halteeinheit 33 mit dem Gehäuse 4 und/oder dem Reflektor 21 verbinden.

Fig. 4 zeigt, dass die zweite Halteeinheit 33 zweite Haltemittel 35 aufweist, wobei die zweiten Haltemittel 35 insbesondere als stegförmige Haltearme ausgebildet sind. Die zweiten Haltemittel 35 können zumindest bereichsweise zueinander beabstandet sein. Die Beabstandung zwischen den zweiten Haltemitteln 35 kann ferner variieren. Ebenfalls kann der eingeschlossene Winkel zwischen zwei unmittelbar benachbarten zweiten Haltemitteln 35 variieren.

Zur Befestigung der Strahlungsquellen 5 kann das zweite Haltemittel 35 Befestigungsmittel 47 aufweisen, die Befestigungsmittel 47 des zweiten Haltemittels 35 können insbesondere korrespondierend zu den Befestigungsmitteln 47 der ersten Haltemittel 25 ausgebildet sein, so dass auf die vorangegangenen Ausführungen verwiesen werden darf.

In Fig. 4 ist dargestellt, dass die zweiten Haltemittel 35 mit einem zweiten Verbindungsbereich 36 der zweiten Halteeinheit 33 verbunden sind. Ausgehend von diesem Verbindungsbereich 36 stehen die zweiten Haltemittel 35 ab. Die zweiten Haltemittel 35 sind mit dem einen Endbereich 38 mit dem Verbindungsbereich 36 verbunden. Die zweiten Haltemittel 35 umfassen ferner einen weiteren freien bzw. nicht-gelagerten Endbereich 39, der wiederum zur Anordnung der Strahlungsquellen 5, insbesondere der weiteren stirnseitigen Endbereiche 37 der Strahlungsquelle 5, vorgesehen ist.

Der Endbereich 38 kann an dem Verbindungsbereich 36 gelagert oder mit diesem fest verbunden sein. Auch kann vorgesehen sein, dass der Verbindungsbereich 36 und der Endbereich 38 einteilig miteinander ausgebildet sind.

Nicht näher dargestellt ist, dass ein zweites Verstellmittel an dem Endbereich 38 angeordnet ist. Dieses zweite Verstellmittel kann insbesondere entsprechend des ersten Verstellmittels 30 ausgebildet sein, so dass auf die Ausführungen zum ersten Verstellmittel 30 verwiesen werden darf

Nicht dargestellt ist, dass die zweiten Haltemittel 35 zumindest bereichsweise auch teleskopierbar ausgebildet sind.

Auch die zweiten Haltemittel 35 können eine unterschiedliche Länge Z aufweisen.

Nicht näher dargestellt ist, dass auch die zweiten Haltemittel 35 Anordnungsbereiche für die Strahlungsquelle(n) 5 aufweisen können. Diese Anordnungsbereiche können wie die Anordnungsbereiche 31 der ersten Halteeinheit 23 ausgebildet sein.

Das zweite Verbindungsmittel 34 ist in dem in Fig. 4 dargestellten Ausführungsbeispiel mehrteilig ausgebildet und weist eine Mehrzahl von entsprechenden Haltestreben auf. Die Haltestreben des zweiten Verbindungsmittels 34 können die zweite Halteeinheit 33 lösbar mit dem Gehäuse 4 und/oder dem Reflektor 21 verbinden.

Fig. 4 zeigt weiter, dass die erste Halteeinheit 23 mit der zweiten Halteeinheit 33 über ein Verbindungsteil 45 verbunden ist. Das Verbindungsteil 45 kann insbesondere langgestreckt ausgebildet sein und verbindet in dem dargestellten Ausführungsbeispiel den ersten Verbindungsbereich 26 mit dem zweiten Verbindungsbereich 36. Das Verbindungsteil 45 ist insbesondere steif und stabil ausgebildet. Die Außenseite des Verbindungsteils 45 kann reflektierend ausgebildet sein.

In Fig. 4 ist dargestellt, dass das Verbindungsteil 45 im Zentrum des Lampenpaketes angeordnet ist und daher von den Strahlungsquellen 5 umschlossen bzw. umgeben wird. Insbesondere steht das Verbindungsteil 45 (in Bezug zur Innenseite 6) nicht über die Strahlungsquellen 5 über.

Besonders bevorzugt ist vorgesehen, dass die zweite Halteeinheit 33 komplementär zur ersten Halteeinheit 23 ausgebildet ist, insbesondere so dass die gewünschte Schrägstellung der Strahlungsquellen 5 erreicht werden kann.

Nicht näher dargestellt ist, dass das erste Verbindungsmittel 24, das zweite Verbindungsmittel 34 und/oder die Haltestreben 46 teleskopierbar und/oder verstellbar ausgebildet sind. Eine solche Verstellung oder Teleskopierung erhöht insbesondere die Flexibilität bzw. die Anpassbarkeit der gesamten Halteeinrichtung 22.

In Fig. 8 ist eine schematische Ansicht der noch nicht ausgerichteten Halteeinrichtung 22 dargestellt. Letztlich sind die jeweiligen Strahlungsquellen 5 noch nicht an die entsprechenden Haltemittel 25, 35 angeordnet.

Fig. 8 zeigt einen dem Anschluss der Strahlungsquellen 5 über Energieversorgungsleitungen 32, die mit einer ersten Energie-Versorgungseinrichtung 41 verbunden sind, in der mehrere Vorschaltgeräte 42 angeordnet sind. Demnach kann ein modularer Aufbau der Halteeinrichtung 22 sichergestellt werden. Der modulare Aufbau kann derart angepasst werden, dass insbesondere unterschiedliche Längen für die Strahlungsquellen 5 ermöglicht werden können.

Zudem zeigt Fig. 8, dass die erste Halteeinheit 23 sowie das erste Verbindungsmittel 24 mit einem ersten Verbindungsabschnitt 40 verbunden sind. Der erste Verbindungsabschnitt 40 ist mit dem Gehäuse 4 und/oder dem Reflektor 21 lösbar verbindbar, was nicht näher dargestellt ist. Beispielsweise kann vorgesehen sein, dass der erste Verbindungsabschnitt 40 zumindest bereichsweise ein Profil zur Anordnung des Reflektors 21 aufweist, der insbesondere als Alu-Blech ausgebildet sein kann. Grundsätzlich sind aber auch weitere Ausführungsformen denkbar.

Der erste Verbindungsabschnitt 40 steht zumindest teilweise in weiteren Ausführungsformen über das Gehäuse ab bzw. über. Dabei kann ferner der erste Verbindungsabschnitt 40 außenseitig eine erste Versorgungseinrichtung 41 aufweisen. Die erste Versorgungseinrichtung 41 umfasst mehrere Vorschaltgeräte 42, wie zuvor erläutert worden ist. Die erste Versorgungseinrichtung 41 ist elektrisch mit dem ersten Verbindungsmittel 24 über die Energieversorgungsleitungen 32 verbunden. Die Energieversorgungsleitungen 32 können durch das Gehäuse 4 geführt werden, wie dies schematisch beispielsweise auch die Fig. 4 zeigt.

Darüber hinaus zeigt Fig. 8, dass die zweite Halteeinheit 33 sowie das zweite Verbindungsmittel 34 mit einem zweiten Verbindungsabschnitt 43 verbunden sind. Der zweite Verbindungsabschnitt 32 kann in weiteren Ausführungsformen ferner auch lösbar mit dem Gehäuse 4 und/oder dem Reflektor 21 verbunden sein. Darüber hinaus kann auch der zweite Verbindungsabschnitt 43 über das Gehäuse 4 in weiteren Ausführungsformen abstehen.

Der erste und zweite Verbindungsabschnitt 40, 43 kann derart ausgebildet sein, dass diese lösbar miteinander formschlüssig und/oder reibschlüssig und/oder kraftschlüssig verbindbar sind. Dazu können die Verbindungsabschnitte 40, 43 entsprechende Verriegelungskonturen oder dergleichen aufweisen. In Fig. 13 ist dargestellt, dass die Verbindungsabschnitte 40, 43 über ihre Stirnseiten verbindbar sind. Entsprechende Verriegelungskonturen sind in Fig. 13 nicht näher dargestellt.

Fig. 13 zeigt, dass zum modularen Aufbau ein weiterer Verbindungsabschnitt 44 vorgesehen ist. Der weitere Verbindungsabschnitt 44 kann lösbar mit dem ersten und/oder zweiten Verbindungsabschnitt 40, 43 formschlüssig und/oder reibschlüssig und/oder kraftschlüssig verbindbar sein. Hierzu kann der weitere Verbindungsabschnitt 44 entsprechende Verriegelungskonturen, die komplementär zu den Verriegelungskonturen der unmittelbar angrenzenden Verbindungsabschnitte ausgebildet sind, aufweisen.

Nicht näher dargestellt ist, dass der erste, zweite und/oder weitere Verbindungsabschnitt 40, 43 und 44 zumindest teilweise in das Innere des Reflektors 21 hineinragen oder an die Innenseite 6 angrenzen kann - oder gegenüber dieser zurückversetzt sein kann.

Je nach Ausführungsform kann vorgesehen sein, dass zwischen 3 bis 25 Strahlungsquellen 5, erste Haltemittel 25 und/oder zweite Haltemittel 35 vorgesehen sind. Die Anzahl der Strahlungsquellen 5 kann insbesondere von der Länge des Reflektors 21, dem behandelten Volumenstrom des Mediums und dergleichen abhängen. In Fig. 9 ist dargestellt, dass zehn Strahlungsquellen 5 vorgesehen sind.

Nicht dargestellt ist, dass die Anzahl der ersten Haltemittel 25 und/oder der zweiten Haltemittel 35 die Anzahl der Strahlungsquellen 5 übersteigt. Es ist daher nicht zwingend erforderlich, dass an jedes Haltemittel 25 eine Strahlungsquelle 5 angeordnet werden muss. Somit kann ein "Überhang" an Haltemitteln 25, 35 vorgesehen sein.

Bei der in Fig. 4 dargestellten Ausführungsform ist vorgesehen, dass die Strahlungsquellen 5 zueinander baugleich ausgebildet sind. Grundsätzlich können auch unterschiedliche Strahlungsquellen 5 gewählt werden, sofern dies vom Nutzer gewünscht wird.

Nicht näher dargestellt ist, dass wenigstens eine, bevorzugt alle, Strahlungsquellen 5 einen Durchmesser D, insbesondere der maximale und/oder der mittlere Durchmesser D, zwischen 1 cm bis 20 cm, insbesondere zwischen 4 cm und 6 cm, aufweisen. Ferner können die Strahlungsquellen 5 eine Länge L zwischen 0,2 bis 10 m, bevorzugt zwischen 1 bis 2 m, aufweisen.

Auch der innere Durchmesser des Reflektors 21 kann variieren und insbesondere zwischen 100 und 1000 cm betragen. Insbesondere liegt der innere Durchmesser zwischen 200 bis 600 cm.

Nicht näher dargestellt ist, dass eine Auswerteeinrichtung zur Erfassung wenigstens einer chemischen und/oder physikalischen Größe vorgesehen sein kann. Insbesondere ist die Auswerteeinrichtung in dem ersten Verbindungsabschnitt 40 und/oder in der ersten Halteeinheit 23 angeordnet. Vorzugsweise weist die Auswerteeinrichtung einen Temperatursensor, einen UV-Sensor und/oder einen Geschwindigkeitssensor auf.

Ebenfalls nicht näher dargestellt ist, dass die Länge Z des ersten Haltemittels 25 und/oder des zweiten Haltemittels 35 zwischen 0,5 • D_{R} bis 0,9 • D_{R} , bevorzugt zwischen 0,1 • D_{R} bis 0,5 • D_{R} , beträgt, wobei D_{R} den inneren Durchmesser des Reflektors 21, insbesondere den maximalen und/oder den mittleren inneren Durchmesser des Reflektors 21, bezeichnet.

### Ausführungsbeispiel 1:

Bei durchgeführten Versuchen unter Verwendung der Bestrahlungsvorrichtung 1, wie sie in der Fig. 3 dargestellt ist, hat sich gezeigt, dass bei einer Bestrahlungsvorrichtung 1 mit einer Leistung von 190 W +/- 10 % und einer Intensität an der Oberfläche der Strahlungsquelle 5 von etwa 4233 W/m² +/- 10 % in Abhängigkeit von verschiedenen Durchströmungsgeschwindigkeiten des Mediumstromes die nachfolgend angegebenen Abtötungsraten der Mikroorganismen erreicht werden können. Die Abtötungsrate der Mikroorganismen ist auch von dem Widerstand der jeweiligen Mikroorganismen gegen UV-C-Strahlung und von der jeweiligen Verweilzeit in dem Gehäuse 4 abhängig.

Das Gehäuse 4 hat eine Länge von 100 cm bei einem Innendurchmesser von 15 cm. Die Strahlungsquelle 5 hat einen Durchmesser von circa 5 cm +/- 10 % bei einer Länge von 15 cm. Die verwendete Strahlungsquelle 5 hat UV-Strahlung bei einer Wellenlänge von 254 nm +/- 2 % bereitgestellt.

Als Medium ist mit Mikroorganismen belastete Luft gewählt worden.

Die nachfolgend angegebene Tabelle stellt die Versuchsergebnisse der erreichten Abtötungsrate für verschiedene Mikroorganismen dar.

| Volumenstrom | Viren | Bakterien | Pilze |
|---|---|---|---|
| 100 m³/h | > 99,999 % | > 99,999 % | 98,72 % |
| 200 m³/h | > 99,999 % | > 99,999 % | 88,68 % |
| 300 m³/h | > 99,999 % | 99,997 % | 76,60 % |
| 400 m³/h | > 99,999 % | 99,96 % | 66,36 % |
| 500 m³/h | > 99,999 % | 99,82 % | 58,17 % |
| 600 m³/h | > 99,999 % | 99,49 % | 51,53 % |
| 700 m³/h | > 99,999 % | 98,92 % | 46,34 % |
| 800 m³/h | 99,999 % | 98,10 % | 41,99 % |
| 900 m³/h | 99,998 % | 97,04 % | 38,38 % |
| 1.000 m³/h | 99,995 % | 95,79 % | 35,32 % |

Die vorgenannte Tabelle verdeutlicht die besonders effiziente Viren- und Bakterieninaktivierung - selbst bei hohen Volumenströmen. Die vorgenannte Tabelle zeigt ferner, dass durch die erfindungsgemäße Ausbildung der Bestrahlungsvorrichtung 1 nicht nur effizient Viren und Bakterien sondern auch Pilze abgetötet werden können.

### Ausführungsbeispiel 2:

Bei durchgeführten Versuchen mit einer Bestrahlungsvorrichtung 1, wie sie in Fig. 4 dargestellt ist, hat sich gezeigt, dass mit einer Leistung von 140 W ± 10% bei einer Intensität an der Oberfläche der Strahlungsquellen 5 von etwa 4233 W/m² ± 10% bei verschiedenen Durchstromgeschwindigkeiten des Mediums deutlich verbesserte Abtötungsraten der Mikroorganismen erreicht werden können.

Dabei hat der Winkel α zwischen der Mittelachse S der jeweiligen Strahlungsquelle 5 und der Mittelachse R des Reflektors zwischen 2° bis 10°, insbesondere 2° ± 20%, betragen.

Das Gehäuse 4 hatte eine Länge von 100 cm bei einem Innendurchmesser von 15 cm. Die verwendeten Strahlungsquellen hatten einen Durchmesser von 5 cm +/-10 % bei einer Länge von 15 cm. Die verwendete Strahlungsquelle 5 hat UV-Strahlung bei einer Wellenlänge von 254 nm +/- 2 % bereitgestellt.

Bei den durchgeführten Versuchen konnte festgestellt werden, dass durch die schräge Anordnung der Strahlungsquellen 5 eine Erhöhung der UV-Strahlungsdosis erreicht werden kann - und zwar im Vergleich zu einem "gerade" ausgerichteten Lampenpaket. Unter einer geraden Ausrichtung ist zu verstehen, dass die Mittelachse S der Strahlungsquellen 5 zumindest im Wesentlichen parallel zur Mittelachse R des Reflektors verläuft.

Die Bestrahlungsdosis konnte in den durchgeführten Versuchen von etwa 600 J/m² bei der geraden Ausrichtung des Lampenpaketes auf wenigstens 800 bis wenigstens 850 J/m² erhöht werden.

Die vorgenannte UV-Strahlungsdosis ist biodosimetrisch - und insbesondere zusätzlich durch ein sogenanntes "internes Raytracing"-Verfahren - ermittelt worden und insbesondere auf Basis des View-Factors bestimmt worden. Letztlich ist die UV-Strahlungsdosis auf für den Fachmann auf bekannte Weise ermittelt worden. Im Hinblick auf die verwendeten Messmethoden darf auf die derzeit noch nicht fertiggestellte DIN(TS) 67506 (in Vorbereitung, Stand Juni 2021) und auf die ISO 15714 verwiesen werden.

Als Medium ist mit Mikroorganismen belastete Luft gewählt worden.

Bei den durchgeführten Versuchen konnte festgestellt werden, dass durch die schräge Anordnung die konstruktive Interferenz auf vorteilhafte Weise zu einer Erhöhung der zu verabreichenden UV-Strahlungsdosis führen kann.

### Bezugszeichenliste:

- 1: Bestrahlungsvorrichtung
- 2: Einlass
- 3: Auslass
- 4: Gehäuse
- 5: Strahlungsquelle
- 6: Innenseite
- 8: Behandlungskammer
- 13: Länge von 4

- 21: Reflektor
- 22: Halteeinrichtung
- 23: erste Halteeinheit
- 24: erstes Verbindungsmittel
- 25: erstes Haltemittel
- 26: Verbindungsbereich
- 27: stirnseitiger Endbereich von 5
- 28: Endbereich von 25
- 29: freier Endbereich von 25
- 30: erstes Verstellmittel
- 31: Anordnungsbereich
- 32: Energieversorgungsleitung(en)
- 33: zweite Halteeinheit
- 34: zweites Verbindungsmittel
- 35: zweites Haltemittel
- 36: zweiter Verbindungsbereich
- 37: weiterer stirnseitiger Endbereich von 5
- 38: Endbereich von 35
- 39: freier Endbereich von 35
- 40: erster Verbindungsabschnitt
- 41: erste Versorgungseinrichtung
- 42: Vorschaltgeräte
- 43: zweiter Verbindungsabschnitt
- 44: weiterer Verbindungsabschnitt
- 45: Verbindungsteil
- 46: Haltestreben
- 47: Befestigungsmittel

- α: Winkel
- β: Winkel zwischen 25
- γ: Winkel zwischen 25
- δ: Winkel zwischen 5

- A₁, A₂: Abstand von 5 zu 6
- S, S₁, S₂: Mittelachse Strahlungsquelle
- R: Mittelachse Reflektor
- D: Durchmesser von 5
- L: Länge von 5
- Z, Z₁, Z₂: Länge erstes bzw. zweites Haltemittel

## Patentansprüche

1. Bestrahlungsvorrichtung (1) zur UV-Bestrahlung, insbesondere UV-C-Bestrahlung, eines die Bestrahlungsvorrichtung (1) durchströmenden Mediums, insbesondere Wasser oder ein gasförmiges Medium, bevorzugt Luft, insbesondere zur Inaktivierung von in dem Medium befindlichen Mikroorganismen, wie Bakterien, Keime, Schimmel und/oder Viren, mit einem einen Einlass (2) und einen Auslass (3) für das Medium aufweisenden zu durchströmenden Gehäuse (4) und einer Mehrzahl von im Inneren des Gehäuses (4) angeordneten, UV-Strahlung emittierenden Strahlungsquellen (5) zur Bestrahlung des das Gehäuse (4) durchströmenden Mediums,
wobei das Gehäuse (4) einen Reflektor (21) aufweist, wobei der Reflektor (21) an der der Strahlungsquelle (5) zugewandten Innenseite (6) zumindest bereichsweise, vorzugsweise vollflächig, reflektierend einen Reflexionsgrad für die von der Strahlungsquelle (5) emittierten UV-Strahlung von wenigstens 0,6 aufweist,
wobei die wenigstens eine Strahlungsquelle (5) mittels einer Halteeinrichtung (22) gehalten und/oder fixiert ist, wobei die Halteeinrichtung (22), vorzugsweise lösbar, mit dem Gehäuse (4) und/oder dem Reflektor (21) verbunden ist,
**dadurch gekennzeichnet,**
**dass** die Halteeinrichtung (22) eine erste Halteeinheit (23) für die Strahlungsquellen (5) aufweist,
**dass** wenigstens zwei Strahlungsquellen (5) derart in dem und/oder an der ersten Halteeinheit (23) angeordnet und/oder gelagert sind, dass die minimalen Abstände (A₁, A₂) der Strahlungsquellen (5) zu der Innenseite des Reflektors (21) sich voneinander unterscheiden, und/oder
**dass** die erste Halteeinheit (23) über ein erstes Verbindungsmittel (24) der Halteeinrichtung (22) mit dem Gehäuse (4) lösbar verbindbar ist, wobei die erste Halteeinheit (23) wenigstens drei zueinander zumindest bereichsweise beabstandete, als stegförmige und/oder langgestreckte Haltearme ausgebildete erste Haltemittel (25) aufweist, wobei die ersten Haltemittel (25) mit einem Endbereich (28) an einem ersten, mit dem Verbindungsmittel (24) verbundenen Verbindungsbereich (26) gehalten sind und wobei die Strahlungsquellen (5), vorzugsweise jeweils an einem stirnseitigen Endbereich (27), mit den ersten Haltemitteln (25), vorzugsweise lösbar und formschlüssig, kraftschlüssig und/oder reibschlüssig, an dem anderen, insbesondere freien, Endbereich (29) der ersten Haltemittel (25) verbunden sind.

2. Bestrahlungsvorrichtung nach Anspruch 1, **dadurch gekennzeichnet, dass** wenigstens zwei Strahlungsquellen (5) derart in dem und/oder an der ersten Halteeinheit (23) angeordnet und/oder gelagert sind, dass die entlang der jeweiligen Längsrichtung der wenigstens zwei Strahlungsquellen (5) verlaufenden Mittelachsen (S, S₁, S₂) miteinander einen Winkel einschließen, und/oder
dass wenigstens zwei Strahlungsquellen (5) derart in dem und/oder an der ersten Halteeinheit (23) angeordnet bzw. gelagert sind, dass die entlang der jeweiligen Längsrichtung der wenigstens zwei Strahlungsquellen (5) verlaufenden Mittelachsen (S, S₁, S₂) miteinander einen Winkel zwischen 1° bis 120°, bevorzugt zwischen 5° bis 90°, weiter bevorzugt zwischen 10° bis 40°einschließen, und/oder
dass wenigstens zwei Strahlungsquellen (5) derart in dem und/oder an der ersten Halteeinheit (23) angeordnet bzw. gelagert sind, dass die minimalen Abstände (A₁, A₂) zu der Innenseite des Reflektors (21) sich voneinander um wenigstens 5%, bevorzugt wenigstens 20%, weiter bevorzugt wenigstens 30%, unterscheiden.

3. Bestrahlungsvorrichtung nach Anspruch 1 oder 2, **dadurch gekennzeichnet, dass** die ersten Haltemittel (25) langgestreckt ausgebildet sind und dass wenigstens zwei erste Haltemittel (25) eine sich voneinander unterscheidende Länge (Z, Z₁, Z₂) aufweisen und/oder dass wenigstens ein erstes Haltemittel (25) wenigstens zwei Anordnungsbereiche (31) zur Verbindung mit der Strahlungsquelle (5) aufweist.

4. Bestrahlungsvorrichtung nach einem der vorhergehenden Ansprüche, **dadurch gekennzeichnet, dass** die erste Halteeinheit (23) derart ausgebildet ist, dass wenigstens zwei zwischen zwei unmittelbar benachbarten ersten Haltemitteln (25) eingeschlossene Winkel (β, γ) zueinander unterschiedlich ausgebildet sind, insbesondere um wenigstens 5%, bevorzugt wenigstens 10%, zueinander.

5. Bestrahlungsvorrichtung nach einem der vorhergehenden Ansprüche, **dadurch gekennzeichnet, dass** die Halteeinrichtung (22) derart ausgebildet ist, dass die Mittelachse (S) der wenigstens einen Strahlungsquelle (5) einen Winkel (α) zu der Mittelachse (R) des Reflektors (21) einschließt.

6. Bestrahlungsvorrichtung nach einem der vorhergehenden Ansprüche, **dadurch gekennzeichnet, dass** der eingeschlossene Winkel (α) zwischen der Mittelachse (S, S₁, S₂) der wenigstens einen Strahlungsquelle (5) und der Mittelachse (R) des Reflektors (21) zwischen arcsin((0,2 • D) / L) und arcsin((4 • D) / L), bevorzugt zwischen arcsin((0,5 • D) / L) und arcsin((3 • D) / L), weiter bevorzugt zwischen arcsin(D / L) und arcsin((2 • D) / L), und/oder zwischen 0,5° bis 15°, bevorzugt zwischen 2° bis 10°, weiter bevorzugt 2° +/- 0,5°, liegt, wobei L die Länge der Strahlungsquelle (5) und D den, insbesondere maximalen, Durchmesser der Strahlungsquelle (5) angibt.

7. Bestrahlungsvorrichtung nach einem der vorhergehenden Ansprüche, **dadurch gekennzeichnet, dass** das erste Haltemittel (25) über ein mit dem Verbindungsbereich (26) verbundenes erstes Verstellmittel (30) verstellbar ist, insbesondere derart, die Schrägstellung der Mittelachse (S) der an dem ersten Haltemittel (25) befestigten Strahlungsquelle (5) in Bezug zu der Mittelachse (R) des Reflektors (21) verstellbar ist.

8. Bestrahlungsvorrichtung nach einem der vorhergehenden Ansprüche, **dadurch gekennzeichnet, dass** die Strahlungsquelle (5) zumindest im Wesentlichen stabförmig, zylinderförmig und/oder langgestreckt ausgebildet ist, insbesondere wobei die Längserstreckung der Strahlungsquelle (5) zumindest im Wesentlichen in Längsrichtung des Gehäuses (4) und/oder des Reflektors (21) verläuft.

9. Bestrahlungsvorrichtung nach einem der vorhergehenden Ansprüche, **dadurch gekennzeichnet, dass** die Mittelachsen (S, S₁, S₂) der Strahlungsquellen (5) und/oder wenigstens zwei Mittelachsen (S, S₁, S₂) der Strahlungsquellen (5) zueinander parallel angeordnet sind, oder
dass alle Mittelachsen (S, S₁, S₂) der Strahlungsquellen (5) zueinander schräg und/oder windschief angeordnet sind.

10. Bestrahlungsvorrichtung nach einem der vorhergehenden Ansprüche, **dadurch gekennzeichnet, dass** die Strahlungsquellen (5) zueinander gleich beabstandet sind und/oder dass wenigstens zwei, insbesondere unmittelbar, benachbarte Strahlungsquellen (5) mit ihrer Mittelachse (S, S₁, S₂) einen unterschiedlichen Winkel (a) zu der Mittelachse (R) des Reflektors (21) einschließen.

11. Bestrahlungsvorrichtung nach einem der vorhergehenden Ansprüche, **dadurch gekennzeichnet, dass** die erste Halteeinheit (23) zur Energiezuführung zu den Strahlungsquellen (5) ausgebildet ist, insbesondere wobei in dem ersten Verbindungsmittel (24), in der ersten Halteeinheit (23) und/oder in den erstem Haltemitteln (25) Energieversorgungsleitungen (32) angeordnet sind.

12. Bestrahlungsvorrichtung nach einem der vorhergehenden Ansprüche, **dadurch gekennzeichnet, dass** eine zweite Halteeinheit (33) vorgesehen ist, wobei die zweite Halteeinheit (33) über wenigstens ein zweites Verbindungsmittel (34) der Halteeinrichtung (22) mit dem Gehäuse (4) lösbar verbindbar ist, wobei die zweite Halteeinheit (33) eine Mehrzahl von zueinander zumindest bereichsweise beabstandeten zweiten Haltemitteln (35), vorzugsweise ausgebildet als, insbesondere stegförmige, Haltearme, aufweist, insbesondere wobei die Strahlungsquelle (5), vorzugsweise an einem weiteren stirnseitigen Endbereich (37), mit dem zweiten Haltemittel (35), vorzugsweise lösbar und formschlüssig, kraftschlüssig und/oder reibschlüssig, verbunden ist und/oder
insbesondere wobei die Strahlungsquelle (5) und/oder die Strahlungsquellen (5) beidseitig an der ersten und der zweiten Halteeinheit (23, 33), insbesondere jeweils in einem stirnseitigen Endbereich (27, 37), gelagert ist/sind.

13. Bestrahlungsvorrichtung nach einem der vorhergehenden Ansprüche, **dadurch gekennzeichnet, dass** die erste Halteeinheit (23), insbesondere der Verbindungsbereich (26), mit der zweiten Halteeinheit (33), insbesondere einem zweiten, mit dem zweiten Verbindungsmittel (34) verbundenen Verbindungsbereich (36) zur Verbindung der zweiten Haltemittel (35) mit dem zweiten Verbindungsbereich (36), über ein langgestrecktes, vorzugsweise steifes, Verbindungsteil verbunden ist.

14. Bestrahlungsvorrichtung nach einem der vorhergehenden Ansprüche, **dadurch gekennzeichnet, dass** die erste Halteeinheit (23) und/oder das erste Verbindungsmittel (24) mit einem ersten Verbindungsabschnitt (40) verbunden sind, insbesondere wobei der erste Verbindungsabschnitt (40) mit dem Gehäuse (4) und/oder dem Reflektor (21) lösbar verbindbar ist und/oder insbesondere wobei der erste Verbindungsabschnitt (40) zumindest teilweise über das Gehäuse (4) absteht und/oder insbesondere wobei an dem ersten Verbindungsabschnitt (40) außenseitig eine erste Versorgungseinrichtung (41), vorzugsweise aufweisende eine Mehrzahl von Vorschaltgeräten (42), anordnenbar und/oder verbindbar ist, wobei, vorzugsweise, die erste Versorgungseinrichtung (41) elektrisch über den ersten Verbindungsabschnitt (40) mit der ersten Halteeinrichtung (22) verbunden ist, und/oder dass die zweite Halteeinheit (33) und/oder das zweite Verbindungsmittel (34) mit einem zweiten Verbindungsabschnitt (43) verbunden sind, insbesondere wobei der zweite Verbindungsabschnitt (43) mit dem Gehäuse (4) und/oder dem Reflektor (21) lösbar verbindbar ist und/oder insbesondere wobei der zweite Verbindungsabschnitt (43) zumindest teilweise über das Gehäuse (4) absteht und/oder insbesondere wobei der erste und der zweite Verbindungsabschnitt (40, 43) lösbar miteinander formschlüssig und/oder reibschlüssig und/oder kraftschlüssig verbindbar und/oder verbunden sind und/oder insbesondere wobei wenigstens ein weiterer Verbindungsabschnitt (44) vorgesehen ist, der lösbar mit dem ersten und/oder zweiten Verbindungsabschnitt (40, 43) formschlüssig und/oder reibschlüssig und/oder kraftschlüssig verbindbar und/oder verbunden ist.

15. Bestrahlungsvorrichtung nach einem der vorhergehenden Ansprüche, **dadurch gekennzeichnet, dass** zwischen 3 bis 25, bevorzugt zwischen 4 bis 15, weiter bevorzugt zwischen 5 bis 10, Strahlungsquellen (5), erste Haltemittel (25) und/oder zweite Haltemittel (35) vorgesehen sind und/oder dass die Strahlungsquellen (5) baugleich ausgebildet sind und/oder
dass wenigstens eine, bevorzugt alle, Strahlungsquelle (5) einen Durchmesser (D) zwischen 1 cm bis 20 cm, bevorzugt zwischen 2 bis 10 cm und weiter bevorzugt zwischen 4 bis 6 cm, aufweist und/oder
dass wenigstens eine, bevorzugt alle, Strahlungsquelle (5) eine Länge (L) zwischen 0,2 m bis 10 m, bevorzugt zwischen 0,5 bis 5 m, weiter bevorzugt zwischen 1 bis 2 m, aufweist und/oder
dass der innere Durchmesser des Reflektors (R) zwischen 100 bis 1000 cm, bevorzugt zwischen 200 bis 600 cm, beträgt und/oder
dass die Länge des ersten Haltemittels (23) und/oder des weiten Haltemittels zwischen 0,05 • D_{R} bis 0,99 • D_{R}, bevorzugt zwischen 0,1 • D_{R} bis 0,5 • D_{R}, beträgt, wobei D_{R} den inneren Durchmesser des Reflektors (21) bezeichnet, und/oder
dass wenigstens eine Strahlungsquelle (5), bevorzugt alle, UV-Strahlung in einem Wellenlängenbereich von wenigstens 240 nm bis 300 nm, bevorzugt in einem Wellenlängenbereich von 250 nm bis 285 nm, weiter bevorzugt von 270 nm bis 280 nm und insbesondere von 254 nm +/- 10 % und/oder von 278 nm +/- 10 %, emittiert.
